# EUROPÄISCHE PATENTSCHRIFT

(11) **EP 0 946 616 B1**
(45) Veröffentlichungstag und Bekanntmachung des Hinweises auf die Patenterteilung: **10.10.2001**
(21) Anmeldenummer: 97954405.3
(22) Anmeldetag: 16.12.1997
(51) Int. Cl.: C08F 210/02, C08F 232/00, C08F 4/60

(54) **VERFAHREN ZUR HERSTELLUNG EINES CYCLOOLEFINCOPOLYMERS**
METHOD FOR PRODUCING A CYCLOOLEFIN COPOLYMER
PROCEDE DE PRODUCTION D'UN COPOLYMERE CYCLO-OLEFINIQUE

(30) Priorität: 17.12.1996 DE 19652338
(43) Veröffentlichungstag der Anmeldung: 06.10.1999
(73) Patentinhaber: Ticona GmbH, 65451 Kelsterbach (DE)
(72) Erfinder: JACOBS, Alexandra, D-65931 Frankfurt am Main (DE); FINK, Gerhard, D-45470 Mülheim (DE); RUCHATZ, Dieter, D-63594 Hasselroth (DE)
(86) Internationale Anmeldenummer: EP9707043
(87) Internationale Veröffentlichungsnummer: WO9827125

(56) Entgegenhaltungen:
- EP-A- 0 671 404
- WO-A-96/40806
- US-A- 5 635 573

## Beschreibung

Die Erfindung betrifft ein Verfahren zur Herstellung von Cycloolefincopolymeren mit hohen Molmassen.

Aus der Literatur ist bekannt, daß mit Metallocen-Aluminoxan-Katalysatorsystemen Cycloolefinhomopolymere und -copolymere hergestellt werden können (EP-A-283 164, EP-A-407 870). Die Polymerisation der Cycloolefine verläuft dabei unter Erhalt der Cyclen und kann in Lösungsmitteln oder in Masse durchgeführt werden. Als Lösungsmittel können Kohlenwasserstoffe eingesetzt werden.

Aus der EP-A-0 671 404 sind Metallocen Katalysatoren mit verbrückten Liganden bekannt.

Cycloolefincopolymere können mit einem hohen Gehalt an Cycloolefin hergestellt werden und besitzen dann eine hohe Glastemperatur. Damit verbunden ist eine hohe thermische Formbeständigkeit, weswegen diese Polymere sich zur Verwendung als thermoplastische Formmassen eignen. Cycloolefincopolymere mit einem niedrigeren Gehalt an Cycloolefin weisen eine geringere Glastemperatur auf. Sie besitzen bei Gebrauchstemperatur eine höhere Duktilität und können elastomere Eigenschaften aufweisen.

Bei Cycloolefincopolymeren, die mittels Metallocentechnologie hergestellt werden, zeigt sich, daß sie relativ niedrige Massenmittel der Molmasse aufweisen. Außerdem entstehen bei der Verwendung von Ethylen als Comonomer häufig teilkristalline Ethylenpolymerisate als Nebenprodukte, welche die Transparenz der Cycloolefincopolymere deutlich beeinträchtigen können.

Die Aufgabe der vorliegenden Erfindung liegt darin, ein Verfahren mit hoher Polymerisationaktivität zur Herstellung von Cycloolefincppolymeren mit höherem Massenmittel der Molmasse bei gleichzeitig hoher Transparenz bereitzustellen.

Die Aufgabe der vorliegenden Erfindung wird durch ein Verfahren zur Herstellung eines Cycloolefincopolymers durch Polymerisation von 0,1 bis 99,9 Gew.-%, bezogen auf die Gesamtmenge der Monomeren, mindestens eines polycyclischen Olefins, 0 bis 99,9 Gew.-%, bezogen auf die Gesamtmenge der Monomeren, mindestens eines monocyclischen Olefins und 0,1 bis 99,9 Gew.-%, bezogen auf die Gesamtmenge der Monomeren, mindestens eines acyclischen 1-Olefins, in Gegenwart eines Katalysatorsystems gelöst.

Die Polymerisation wird im flüssigen Cycloolefin selbst oder in Cycloolefin-Lösung durchgeführt, wobei zweckmäßigerweise der Druck oberhalb 1 bar liegt.

Das für das erfindungsgemäße Verfahren zu verwendende Katalysatorsystem ënthält mindestens einen Metall-Koordinations-Komplex, der ein Metall der Gruppe 3 bis 10 oder der Lanthaniden-Reihe des Periodensystems der Elemente und ein an das Metall koordiniertes konjugiertes π-System enthält. Das an das Metall koordinierte konjugierte π-System kann durch eine Gruppe substituiert sein, die ebenfalls an das Metallatom koordiniert ist und die gespannte Geometrie des Metall-Koordinations-Komplexes bewirkt. Vergleichbare Strukturen sind in EP-A-416 815, in EP-A-418 044 und in EP-A-698 618 beschrieben.

Bei dem für das erfindungsgemäße Verfahren zu verwendenden Metall-Koordinations-Komplex handelt es sich um eine Verbindung der Formel (I) 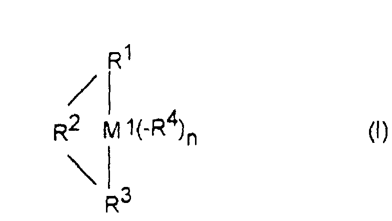 wobei
- M¹: ein Metall der Gruppe 3 bis 10 oder der Lanthaniden-Reihe des Periodensystems der Elemente ist,
- R¹: ein delokalisiertes acyclisches n-System wie C₄-C₂₀-Alkenyl, C₄-C₂₀-Alkinyl, C₃-C₂₀-Allyl, C₄-C₂₀-Alkadienyl, C₄-C₂₀-Polyenyl oder vergleichbare Strukturen, die bis zu 5 Heteroatomen enthalten können, oder ein unsubstituiertes oder substituiertes delokalisiertes C₅-C₄₀-cylisches π-System oder vergleichbare Strukturen, die bis zu 5 Heteroatomen enthalten können, bedeutet,
- R²: eine ein- oder mehrgliedrige Brücke ist, welche die Reste R¹ und R³ verknüpft und mindestens ein Atom der Gruppe 14 des Periodensystems der Elemente oder mindestens ein Bor-Atom enthält und ein oder mehrere Schwefel- oder Sauerstoffatome enthalten kann und mit R¹ ein kondensiertes Ringsystem bilden kann,
- R³: einen anionischen oder nichtionischen Liganden bedeutet, der an M¹ koordiniert ist und ein oder mehrere Stickstoff-, Phosphor-, Sauerstoff- und/ oder Schwefelatome enthält und mit R² ein kondensiertes Ringsystem bilden kann, und
- R⁴: ein anionischer oder nichtionischer Ligand ist, wobei n = 0, 1, 2, 3 oder 4 in Abhängigkeit von der Valenz von M bedeutet.

Das für das erfindungsgemäße Verfahren zu verwendende Voraktivierte Katalysatorsystem enthält ßerdem einen oder mehrere Cokatalysatoren.

Das für das erfindungsgemäße Verfahren zu verwendende voraktivierte Katalysatorsystem ist ein hochaktiver Katalysator für die Olefinpolymerisation. Bevorzugt werden ein Metall-Koordinations-Komplex und ein Cokatalysator eingesetzt. Es können auch Mischungen von zwei oder mehr Metall-Koordinations-Komplexen verwendet werden, insbesondere zur Herstellung von Reaktorblends oder von Polyolefinen mit breiter oder multimodaler Molmassenverteilung.

Bevorzugt ist ein Metall-Koordinations-Komplex, der ein Metall der Gruppe 4 oder der Lanthaniden-Reihe des Periodensystems der Elemente enthält. Außerdem bevorzugt ist ein Metall-Koordinations-Komplex, der ein delokalisiertes cyclisches η⁵-koordiniertes π-System enthält. Bevorzugt sind delokalisierte cyclische π-Systeme wie Cyclopentadienyl, Indenyl, Fluorenyl oder substituiertes Cyclopentadienyl, substituiertes Indenyl oder substituiertes Fluorenyl oder vergleichbare Strukturen, die bis zu 5 Heteroatomen enthalten können. Dabei können eines oder mehrere der Atome des delokalisierten cyclischen π-Systems substituiert sein, wobei die Substituenten gleich oder verschieden sein können und neben Wasserstoff, Atome der Gruppe 14 des Periodensystems der Elemente und/ oder ein oder mehrere Heteroatome wie der Gruppe 15, 16, 17 des Periodensystems der Elemente enthalten können. Zwei oder mehrere der Substituenten können einen Ring bilden. Beispiele für substituierte delokalisierte cyclische π-Systeme sind Methylcyclopentadienyl, Ethylcyclopentadienyl, Isopropylcyclopentadienyl, *t*-Butylcyclopentadienyl, Dimethylcyclopentadienyl, Diethylcyclopentadienyl, Diisopropylcyclopentadienyl, Di-*t*-butylcyclopentadienyl Tetramethylcyclopentadienyl.

Besonders bevorzugt handelt es sich bei dem für des erfindungsgemäße Verfahren zu verwendenden Metall-Koordinations-Komplex um eine Verbindung der Formel (la): 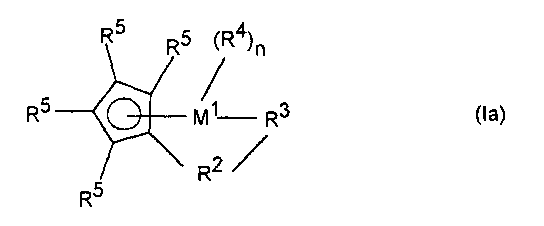 wobei
- M¹: ein Metall der Gruppe 4 oder der Lanthaniden-Reihe des Periodensystems der Elemente ist,
- R²: eine ein- oder mehrgliedrige Brücke ist, welche das η⁵-koordinierte cyclische π-System und R³ verknüpft, und bedeutet vorzugsweise 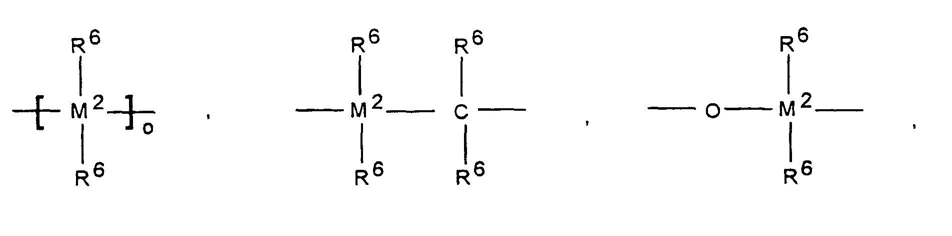 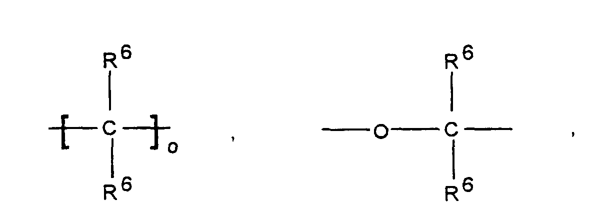 =BR⁶, =AIR⁶, -Ge-, -Sn-, -O-, -S-, =SO, =SO₂, =NR⁶, =CO, =PR⁶, oder =P(O)R⁶, wobei R⁶ gleich oder verschieden sind und ein Wasserstoffatom, ein Halogenatom, eine C₁-C₄₀-kohlenstoffhaltige Gruppe wie eine C₁-C₁₀-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe, -SiR⁷₃, -NR⁷₂, -Si(OR⁷)₃,-Si(SR⁷)₃ oder -PR⁷₂ bedeuten,
worin R⁷ gleich oder verschieden ein Halogenatom, eine C₁-C₁₀-Alkylgruppe oder eine C₆-C₁₀-Arylgruppe sind oder ein Ringsystem bilden, wobei o ≥ 1 ist, und
- M²: Silicium, Germanium oder Zinn ist,
- R³: O, S, NR⁸, PR⁸ oder einen neutralen 2-Elektronen-Donor-Liganden bedeutet, ausgewählt aus der Gruppe OR⁸, SR⁸, NR⁸₂, PR⁸₂, wobei R⁸ ein Wasserstoffatom, ein Halogenatom, eine C₁-C₄₀-kohlenstoffhaltige Gruppe wie eine C₁-C₈-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe, -SiR⁹₃, -NR⁹₂, -Si(OR⁹)₃,-Si(SR⁹)₃ oder -PR⁹₂ bedeuten, worin R⁹ gleich oder verschieden ein Halogenatom, eine C₁-C₁₀-Alkylgruppe oder eine C₆-C₁₀-Arylgruppe sind,
- R⁴: gleich oder verschieden sind und ein Wasserstoffatom, eine C₁-C₄₀-kohlenstoffhaltige Gruppe wie eine C₁-C₁₀-Alkyl-, eine C₁-C₁₀-Alkoxy-, eine C₆-C₁₀-Aryl-, eine C₆-C₂₅-Aryloxy-, eine C₂-C₁₀-Alkenyl-, eine C₇-C₄₀-Arylalkyl- oder eine C₇-C₄₀-Arylalkenylgruppe, eine OH-Gruppe, ein Halogenatom oder NR¹⁰₂, worin R¹⁰ ein Halogenatom, eine C₁-C₁₀-Alkylgruppe oder eine C₆-C₁₀-Arylgruppe ist, bedeuten, oder R⁴ zusammen mit den sie verbindenden Atomen ein Ringsystem bilden, wobei n = 1 oder 2 ist,
- R⁵: gleich oder verschieden sind und ein Wasserstoffatom, ein Halogenatom, eine C₁-C₄₀-kohlenstoffhaltige Gruppe wie eine C₁-C₁₀-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, oder eine C₈-C₄₀-Arylalkenylgruppe, -SiR¹¹₃, -NR¹¹₂,-Si(OR¹¹)₃,-Si(SR¹¹)₃ oder -PR¹¹₂ bedeuten, worin R¹¹ gleich oder verschieden ein Halogenatom, eine C₁-C₁₀-Alkylgruppe oder eine C₆-C₁₀-Aryigruppe sind oder ein Ringsystem bilden, oder zwei oder mehr benachbarte Substituenten R⁵ zusammen mit den sie verbindenden Atomen ein Ringsystem bilden, welches bevorzugt 4 bis 40, besonders bevorzugt 6 bis 20 Kohlenstoffatome enthält.

Ganz besonders bevorzugt handelt es sich bei dem für das erfindungsgemäße Verfahren zu verwendenden Metall-Koordinations-Komplex um eine Verbindung der Formel (Ib): 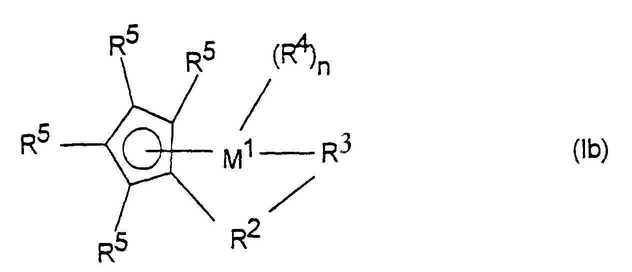 wobei
- M¹: Titan bedeutet,
- R²: eine ein-, zwei- oder dreigliedrige Brücke ist, welche das η⁵-koordinierte cyclische π-System und R³ verknüpft, und bedeutet vorzugsweise 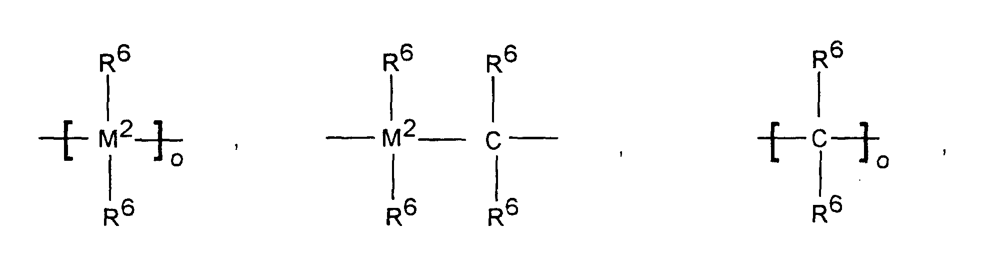 wobei R⁶ gleich oder verschieden sind und ein Wasserstoffatom, eine C₁-C₄₀-kohlenstoffhaltige Gruppe wie eine C₁-C₁₀-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe bedeutet, wobei o = 1, 2 oder 3 ist, und
- M²: Silicium ist,
- R³: NR⁸ ist, wobei R⁸ ein Wasserstoffatom, eine C₁-C₄₀-kohlenstoffhaltige Gruppe wie eine C₁-C₈-Alkylgruppe, die halogeniert sein kann, C₁-C₁₀-Alkoxygruppe, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe ist,
- R⁴: gleich oder verschieden sind und ein Wasserstoffatom, eine C₁-C₄₀-kohlenstoffhaltige Gruppe wie eine C₁-C₁₀-Alkyl-, eine C₁-C₁₀-Alkoxy-, eine C₆-C₁₀-Aryl-, eine C₆-C₂₅-Aryloxy-, eine C₂-C₁₀-Alkenyl-, eine C₇-C₄₀-Arylalkyl- oder eine C₇-C₄₀-Arylalkenylgruppe, eine OH-Gruppe, ein Halogenatom oder NR¹⁰₂, worin R¹⁰ ein Halogenatom, eine C₁-C₁₀-Alkylgruppe oder eine C₆-C₁₀-Arylgruppe sind, bedeuten, oder zusammen mit den sie verbindenden Atomen ein Ringsystem bilden,
- R⁵: gleich oder verschieden sind und ein Wasserstoffatom, eine C₁-C₁₀-Alkyl-oder Trimethylsilyl-Gruppe bedeuten oder zwei der Substituenten R⁵ mit dem sie verbindenden Cyclopentadienyl-System einen sechsgliedrigen aromatischen kondensierten Ring bilden.

Beispiele für die erfindungsgemäß zu verwendenden Metall-Koordinations-Komplexe sind:
(Methylamido)(η⁵-cyclopentadienyl)dimethylsilanzirkondichlorid
(Methylamido)(η⁵-cyclopentadienyl)dimethylsilanzirkonbis(diethylamid)
(Ethylamido)(η⁵-cyclopentadienyl)dimethylsilanzirkondichlorid
(Ethylamido)(η⁵-cyclopentadienyl)dimethylsilanzirkonbis(diethylamid)
(Isopropylamido)(η⁵-cyclopentadienyl)dimethylsilanzirkondichlorid
(Isopropylamido)(η⁵-cyclopentadienyl)dimethylsilanzirkonbis(diethylamid)
(t-Butylamido)(η⁵-cyclopentadienyl)dimethylsilanzirkondichlorid
(t-Butylamido)(η⁵-cyclopentadienyl)dimethylsilanzirkonbis(diethylamid)
(Phenylamido)(η⁵-cyclopentadienyl)dimethylsilanzirkondichlorid
(Phenylamido)(η⁵-cyclopentadienyl)dimethylsilanzirkonbis(diethylamid)
(Cyclohexylamido)(η⁵-cyclopentadienyl)dimethylsilanzirkondichlorid
(Cyclohexylamido)(η⁵-cyclopentadienyl)dimethylsilanzirkonbis(diethylamid)
(Methylamido)(η⁵-cyclopentadienyl)dimethylsilantitandichlorid
(Methylamido)(η⁵-cyclopentadienyl)dimethylsilantitandibromid
(Methylamido)(η⁵-cyclopentadienyl)dimethylsilantitanbis(dimethylamid)
(Methylamido)(η⁵-cyclopentadienyl)dimethylsilantitanbis(diethylamid)
(Ethylamido)(η⁵-cyclopentadienyl)dimethylsilantitandichlorid
(Ethylamido)(η⁵-cyclopentadienyl)dimethylsilantitandibromid
(Ethylamido)(η⁵-cyclopentadienyl)dimethylsilantitanbis(dimethylamid)
(Ethylamido)(η⁵-cyclopentadienyl)dimethylsilantitanbis(diethylamid)
(Isopropylamido)(η⁵-cyclopentadienyl)dimethylsilantitandichlorid
(Isopropylamido)(η⁵-cyclopentadienyl)dimethylsilantitandibromid
(Isopropylamido)(η⁵-cyclopentadienyl)dimethylsilantitanbis(dimethylamid)
(Isopropylamido)(η⁵-cyclopentadienyl)dimethylsilantitanbis(diethylamid)
(t-Butylamido)(η⁵-cyclopentadienyl)dimethylsilantitandichlorid
(t-Butylamido)(η⁵-cyclopentadienyl)dimethylsilantitandibromid
(t-Butylamido)(η⁵-cyclopentadienyl)dimethylsilantitanbis(dimethylamid)
(t-Butylamido)(η⁵-cyclopentadienyl)dimethylsilantitanbis(diethylamid)
(Phenylamido)(η⁵-cyclopentadienyl)dimethylsilantitandichlorid
(Phenylamido)(η⁵-cyclopentadienyl)dimethylsilantitandibromid
(Phenylamido)(η⁵-cyclopentadienyl)dimethylsilantitanbis(dimethylamid)
(Phenylamido)(η⁵-cyclopentadienyl)dimethylsilantitanbis(diethylamid)
(Cyclohexylamido)(η⁵-cyclopentadienyl)dimethylsilantitandichlorid
(Cyclohexylamido)(η⁵-cyclopentadienyl)dimethylsilantitandibromid
(Cyclohexylamido)(η⁵-cyclopentadienyl)dimethylsilantitanbis(dimethylamid)
(Cyclohexylamido)(η⁵-cyclopentadienyl)dimethylsilantitanbis(diethylamid)
(Methylamido)(3-methyl-η⁵-cyclopentadienyl)dimethylsilanzirkondichlorid
(Methylamido)(3-methyl-η⁵-cyclopentadienyl)dimethylsilanzirkonbis(diethylamid)
(Ethylamido)(3-methyl-η⁵-cyclopentadienyl)dimethylsilanzirkondichlorid
(Ethylamido)(3-methyl-η⁵-cyclopentadienyl)dimethylsilanzirkonbis(diethylamid)
(Isopropylamido)(3-methyl-η⁵-cyclopentadienyl)dimethylsilanzirkondichlorid
(Isopropylamido)(3-methyl-η⁵-cyclopentadienyl)dimethylsilanzirkonbis(diethylamid)
(t-Butylamido)(3-methyl-η⁵-cyclopentadienyl)dimethylsilanzirkondichlorid
(t-Butylamido)(3-methyl-η⁵-cyclopentadienyl)dimethylsilanzirkonbis(diethylamid)
(Phenylamido)(3-methyl-η⁵-cyclopentadienyl)dimethylsilanzirkondichlorid
(Phenylamido)(3-methyl-η⁵-cyclopentadienyl)dimethylsilanzirkonbis(diethylamid)
(Cyclohexylamido)(3-methyl-η⁵-cyclopentadienyl)dimethylsilanzirkondichlorid
(Cyclohexylamido)(3-methyl-η⁵-cyclopentadienyl)dimethylsilanzirkonbis(diethylamid)
(Methylamido)(3-methyl-η⁵-cyclopentadienyl)dimethylsilantitandichlorid
(Methylamido)(3-methyl-η⁵-cyclopentadienyl)dimethylsilantitandibromid
(Methylamido)(3-methyl-η⁵-cyclopentadienyl)dimethylsilantitanbis(dimethylamid)
(Methylamido)(3-methyl-η⁵-cyclopentadienyl)dimethylsilantitanbis(diethylamid)
(Ethylamido)(3-methyl-η⁵-cyclopentadienyl)dimethylsilantitandichlorid
(Ethylamido)(3-methyl-η⁵-cyclopentadienyl)dimethylsilantitandibromid
(Ethylamido)(3-methyl-η⁵-cyclopentadienyl)dimethylsilantitanbis(dimethylamid)
(Ethylamido)(3-methyl-η⁵-cyclopentadienyl)dimethylsilantitanbis(diethylamid)
(Isopropylamido)(3-methyl-η⁵-cyclopentadienyl)dimethylsilantitandichlorid
(Isopropylamido)(3-methyl-η⁵-cyclopentadienyl)dimethylsilantitandibromid
(Isopropylamido)(3-methyl-η⁵-cyclopentadienyl)dimethylsilantitanbis(dimethylamid)
(Isopropylamido)(3-methyl-η⁵-cyclopentadienyl)dimethylsilantitanbis(diethylamid)
(t-Butylamido)(3-methyl-η⁵-cyclopentadienyl)dimethylsilantitandichlorid
(t-Butylamido)(3-methyl-η⁵-cyclopentadienyl)dimethylsilantitandibromid
(t-Butylamido)(3-methyl-η⁵-cyclopentadienyl)dimethylsilantitanbis(dimethylamid)
(t-Butylamido)(3-methyl-η⁵-cyclopentadienyl)dimethylsilantitanbis(diethylamid)
(Phenylamido)(3-methyl-η⁵-cyclopentadienyl)dimethylsilantitandichlorid
(Phenylamido)(3-methyl-η⁵-cyclopentadienyl)dimethylsilantitandibromid
(Phenylamido)(3-methyl-η⁵-cyclopentadienyl)dimethylsilantitanbis(dimethylamid)
(Phenylamido)(3-methyl-η⁵-cyclopentadienyl)dimethylsilantitanbis(diethylamid)
(Cyclohexylamido)(3-methyl-η⁵-cyclopentadienyl)dimethylsilantitandichlorid
(Cyclohexylamido)(3-methyl-η⁵-cyclopentadienyl)dimethylsilantitandibromid
(Cyclohexylamido)(3-methyl-η⁵-cyclopentadienyl)dimethylsilantitanbis(dimethylamid)
(Cyclohexylamido)(3-methyl-η⁵-cyclopentadienyl)dimethylsilantitanbis(diethylamid)
(Methylamido)(3-ethyl-η⁵-cyclopentadienyl)dimethylsilanzirkondichlorid
(Methylamido)(3-ethyl-η⁵-cyclopentadienyl)dimethylsilanzirkonbis(diethylamid)
(Ethylamido)(3-ethyl-η⁵-cyclopentadienyl)dimethylsilanzirkondichlorid
(Ethylamido)(3-ethyl-η⁵-cyclopentadienyl)dimethylsilanzirkonbis(diethylamid)
(Isopropylamido)(3-ethyl-η⁵-cyclopentadienyl)dimethylsilanzirkondichlorid
(Isopropylamido)(3-ethyl-η⁵-cyclopentadienyl)dimethylsilanzirkonbis(diethylamid)
(t-Butylamido)(3-ethyl-η⁵-cyclopentadienyl)dimethylsilanzirkondichlorid
(t-Butylamido)(3-ethyl-η⁵-cyclopentadienyl)dimethylsilanzirkonbis(diethylamid)
(Phenylamido)(3-ethyl-η⁵-cyclopentadienyl)dimethylsilanzirkondichlorid
(Phenylamido)(3-ethyl-η⁵-cyclopentadienyl)dimethylsilanzirkonbis(diethylamid)
(Cyclohexylamido)(3-methyl-η⁵-cyclopentadienyl)dimethylsilanzirkondichlorid
(Cyclohexylamido)(3-ethyl-η⁵-cyclopentadienyl)dimethylsilanzirkonbis(diethylamid)
(Methylamido)(3-ethyl-η⁵-cyclopentadienyl)dimethylsilantitandichlorid
(Methylamido)(3-ethyl-η⁵-cyclopentadienyl)dimethylsilantitandibromid
(Methylamido)(3-ethyl-η⁵-cyclopentadienyl)dimethylsilantitanbis(dimethylamid)
(Methylamido)(3-ethyl-η⁵-cyclopentadienyl)dimethylsilantitanbis(diethylamid)
(Ethylamido)(3-ethyl-η⁵-cyclopentadienyl)dimethylsilantitandichlorid
(Ethylamido)(3-ethyl-η⁵-cyclopentadienyl)dimethylsilantitandibromid
(Ethylamido)(3-ethyl-η⁵-cyclopentadienyl)dimethylsilantitanbis(dimethylamid)
(Ethylamido)(3-ethyl-η⁵-cyclopentadienyl)dimethylsilantitanbis(diethylamid)
(Isopropylamido)(3-ethyl-η⁵-cyclopentadienyl)dimethylsilantitandichlorid
(Isopropylamido)(3-ethyl-η⁵-cyclopentadienyl)dimethylsilantitandibromid
(Isopropylamido)(3-ethyl-η⁵-cyclopentadienyl)dimethylsilantitanbis(dimethylamid)
(Isopropylamido)(3-ethyl-η⁵-cyclopentadienyl)dimethylsilantitanbis(diethylamid)
(t-Butylamido)(3-ethyl-η⁵-cyclopentadienyl)dimethylsilantitandichlorid
(t-Butylamido)(3-ethyl-η⁵-cyclopentadienyl)dimethylsilantitandibromid
(t-Butylamido)(3-ethyl-η⁵-cyclopentadienyl)dimethylsilantitanbis(dimethylamid)
(t-Butylamido)(3-ethyl-η⁵-cyclopentadienyl)dimethylsilantitanbis(diethylamid)
(Phenylamido)(3-ethyl-η⁵-cyclopentadienyl)dimethylsilantitandichlorid
(Phenylamido)(3-ethyl-η⁵-cyclopentadienyl)dimethylsilantitandibromid
(Phenylamido)(3-ethyl-η⁵-cyclopentadienyl)dimethylsilantitanbis(dimethylamid)
(Phenylamido)(3-ethyl-η⁵-cyclopentadienyl)dimethylsilantitanbis(diethylamid)
(Cyclohexylamido)(3-methyl-η⁵-cyclopentadienyl)dimethylsilantitandichlorid
(Cyclohexylamido)(3-ethyl-η⁵-cyclopentadienyl)dimethylsilantitandibromid
(Cyclohexylamido)(3-ethyl-η⁵-cyclopentadienyl)dimethylsilantitanbis(dimethylamid)
(Cyclohexylamido)(3-ethyl-η⁵-cyclopentadienyl)dimethylsilantitanbis(diethylamid)
(Methylamido)(tetramethyl-η⁵-cyclopentadienyl)dimethylsilanzirkondichlorid
(Methylamido)(tetramethyl-η⁵-cyclopentadienyl)dimethylsilanzirkonbis(diethylamid)
(Ethylamido)(tetramethyl-η⁵-cyclopentadienyl)dimethylsilanzirkondichlorid
(Ethylamido)(tetramethyl-η⁵-cyclopentadienyl)dimethylsilanzirkonbis(diethylamid)
(Isopropylamido)(tetramethyl-η⁵-cyclopentadienyl)dimethylsilanzirkondichlorid
(Isopropylamido)(tetramethyl-η⁵-cyclopentadienyl)dimethylsilanzirkonbis(diethylamid)
(t-Butylamido)(tetramethyl-η⁵-cyclopentadienyl)dimethylsilanzirkondichlorid
(t-Butylamido)(tetramethyl-η⁵-cyclopentadienyl)dimethylsilanzirkonbis(diethylamid)
(Phenylamido)(tetramethyl-η⁵-cyclopentadienyl)dimethylsilanzirkondichlorid
(Phenylamido)(tetramethyl-η⁵-cyclopentadienyl)dimethylsilanzirkonbis(diethylamid)
(Cyclohexylamido)(tetramethyl-η⁵-cyclopentadienyl)dimethylsilanzirkondichlorid
(Cyclohexylamido)(tetramethyl-η⁵-cyclopentadienyl)dimethylsilanzirkonbis(diethylamid)
(Methylamido)(tetramethyl-η⁵-cyclopentadienyl)dimethylsilantitandichlorid
(Methylamido)(tetramethyl-η⁵-cyclopentadienyl)dimethylsilantitandibromid
(Methylamido)(tetramethyl-η⁵-cyclopentadienyl)dimethylsilantitanbis(dimethylamid)
(Methylamido)(tetramethyl-η⁵-cyclopentadienyl)dimethylsilantitanbis(diethylamid)
(Ethylamido)(tetramethyl-η⁵-cyclopentadienyl)dimethylsilantitandichlorid
(Ethylamido)(tetramethyl-η⁵-cyclopentadienyl)dimethylsilantitandibromid
(Ethylamido)(tetramethyl-η⁵-cyclopentadienyl)dimethylsilantitanbis(dimethylamid)
(Ethylamido)(tetramethyl-η⁵-cyclopentadienyl)dimethylsilantitanbis(diethylamid)
(Isopropylamido)(tetramethyl-η⁵-cyclopentadienyl)dimethylsilantitandichlorid
(Isopropylamido)(tetramethyl-η⁵-cyclopentadienyl)dimethylsilantitandibromid
(Isopropylamido)(tetramethyl-η⁵-cyclopentadienyl)dimethylsilantitanbis(dimethylamid)
(Isopropylamido)(tetramethyl-η⁵-cyclopentadienyl)dimethylsilantitanbis(diethylamid) (t-Butylamido)(tetramethyl-η⁵-cyclopentadienyl)dimethylsilantitandichlorid
(t-Butylamido)(tetramethyl-η⁵-cyclopentadienyl)dimethylsilantitandibromid
(t-Butylamido)(tetramethyl-η⁵-cyclopentadienyl)dimethylsilantitanbis(dimethylamid) (t-Butylamido)(tetramethyl-η⁵-cyclopentadienyl)dimethylsilantitanbis(diethylamid)
(Phenylamido)(tetramethyl-η⁵-cyclopentadienyl)dimethylsilantitandichlorid
(Phenylamido)(tetramethyl-η⁵-cyclopentadienyl)dimethylsilantitandibromid
(Phenylamido)(tetramethyl-η⁵-cyclopentadienyl)dimethylsilantitanbis(dimethylamid) (Phenylamido)(tetramethyl-η⁵-cyclopentadienyl)dimethylsilantitanbis(diethylamid)
(Cyclohexylamido)(tetramethyl-η⁵-cyclopentadienyl)dimethylsilantitandichlorid
(Cyclohexylamido)(tetramethyl-η⁵-cyclopentadienyl)dimethylsilantitandibromid
(Cyclohexylamido)(tetramethyl-η⁵-cyclopentadienyl)dimethylsilantitanbis(dimethylamid)
(Cyclohexylamido)(tetramethyl-η⁵-cyclopentadienyl)dimethylsilantitanbis(diethylamid)
(Methylamido)(1-η⁵-indenyl)dimethylsilanzirkondichlorid
(Methylamido)(1-η⁵-indenyl)dimethylsilanzirkonbis(diethylamid)
(Ethylamido)(1-η⁵-indenyl)dimethylsilanzirkondichlorid
(Ethylamido)(1-η⁵-indenyl)dimethylsilanzirkonbis(diethylamid)
(Isopropylamido)(1-η⁵-indenyl)dimethylsilanzirkondichlorid
(Isopropylamido)(1-η⁵-indenyl)dimethylsilanzirkonbis(diethylamid)
(t-Butylamido)(1-η⁵-indenyl)dimethylsilanzirkondichlorid
(t-Butylamido)(1-η⁵-indenyl)dimethylsilanzirkonbis(diethylamid)
(Phenylamido)(1-η⁵-indenyl)dimethylsilanzirkondichlorid
(Phenylamido)(1-η⁵-indenyl)dimethylsilanzirkonbis(diethylamid)
(Cyclohexylamido)(1-η⁵-indenyl)dimethylsilanzirkondichlorid
(Cyclohexylamido)(1-η⁵-indenyl)dimethylsilanzirkonbis(diethylamid)
(Methylamido)(1-η⁵-indenyl)dimethylsilantitandichlorid
(Methylamido)(1-η⁵-indenyl)dimethylsilantitandibromid
(Methylamido)(1-η⁵-indenyl)dimethylsilantitanbis(dimethylamid)
(Methylamido)(1-η⁵-indenyl)dimethylsilantitanbis(diethylamid)
(Ethylamido)(1-η⁵-indenyl)dimethylsilantitandichlorid
(Ethylamido)(1-η⁵-indenyl)dimethylsilantitandibromid
(Ethylamido)(1-η⁵-indenyl)dimethylsilantitanbis(dimethylamid)
(Ethylamido)(1-η⁵-indenyl)dimethylsilantitanbis(diethylamid)
(Isopropylamido)(1-η⁵-indenyl)dimethylsilantitandichlorid
(Isopropylamido)(1-η⁵-indenyl)dimethylsilantitandibromid
(Isopropylamido)(1-η⁵-indenyl)dimethylsilantitanbis(dimethylamid)
(Isopropylamido)(1-η⁵-indenyl)dimethylsilantitanbis(diethylamid)
(t-Butylamido)(1-η⁵-indenyl)dimethylsilantitandichlorid
(t-Butylamido)(1-η⁵-indenyl)dimethylsilantitandibromid
(t-Butylamido)(1-η⁵-indenyl)dimethylsilantitanbis(dimethylamid)
(t-Butylamido)(1-η⁵-indenyl)dimethylsilantitanbis(diethylamid)
(Phenylamido)(1-η⁵-indenyl)dimethylsilantitandichlorid
(Phenylamido)(1-η⁵-indenyl)dimethylsilantitandibromid
(Phenylamido)(1-η⁵-indenyl)dimethylsilantitanbis(dimethylamid)
(Phenylamido)(1-η⁵-indenyl)dimethylsilantitanbis(diethylamid)
(Cyclohexylamido)(1-η⁵-indenyl)dimethylsilantitandichlorid
(Cyclohexylamido)(1-η⁵-indenyl)dimethylsilantitandibromid
(Cyclohexylamido)(1-η⁵-indenyl)dimethylsilantitanbis(dimethylamid)
(Cyclohexylamido)(1-η⁵-indenyl)dimethylsilantitanbis(diethylamid)
(Methylamido)(9-η⁵-fluorenyl)dimethylsilanzirkondichlorid
(Methylamido)(9-η⁵-fluorenyl)dimethylsilanzirkonbis(diethylamid)
(Ethylamido)(9-η⁵-fluorenyl)dimethylsilanzirkondichlorid
(Ethylamido)(9-η⁵-fluorenyl)dimethylsilanzirkonbis(diethylamid)
(Isopropylamido)(9-η⁵-fluorenyl)dimethylsilanzirkondichlorid
(Isopropylamido)(9-η⁵-fluorenyl)dimethylsilanzirkonbis(diethylamid)
(t-Butylamido)(9-η⁵-fluorenyl)dimethylsilanzirkondichlorid
(t-Butylamido)(9-η⁵-fluorenyl)dimethylsilanzirkonbis(diethylamid)
(Phenylamido)(9-η⁵-fluorenyl)dimethylsilanzirkondichlorid
(Phenylamido)(9-η⁵-fluorenyl)dimethylsilanzirkonbis(diethylamid)
(Cyclohexylamido)(9-η⁵-fluorenyl)dimethylsilanzirkondichlorid
(Cyclohexylamido)(9-η⁵-fluorenyl)dimethylsilanzirkonbis(diethylamid)
(Methylamido)(9-η⁵-fluorenyl)dimethylsilantitandichlorid
(Methylamido)(9-η⁵-fluorenyl)dimethylsilantitandibromid
(Methylamido)(9-η⁵-fluorenyl)dimethylsilantitanbis(dimethylamid)
(Methylamido)(9-η⁵-fluorenyl)dimethylsilantitanbis(diethylamid)
(Ethylamido)(9-η⁵-fluorenyl)dimethylsilantitandichlorid
(Ethylamido)(9-η⁵-fluorenyl)dimethylsilantitandibromid
(Ethylamido)(9-η⁵-fluorenyl)dimethylsilantitanbis(dimethylamid)
(Ethylamido)(9-η⁵-fluorenyl)dimethylsilantitanbis(diethylamid)
(Isopropylamido)(9-η⁵-fluorenyl)dimethylsilantitandichlorid
(Isopropylamido)(9-η⁵-fluorenyl)dimethylsilantitandibromid
(Isopropylamido)(9-η⁵-fluorenyl)dimethylsilantitanbis(dimethylamid)
(Isopropylamido)(9-η⁵-fluorenyl)dimethylsilantitanbis(diethylamid)
(t-Butylamido)(9-η⁵-fluorenyl)dimethylsilantitandichlorid
(t-Butylamido)(9-η⁵-fluorenyl)dimethylsilantitandibromid
(t-Butylamido)(9-η⁵-fluorenyl)dimethylsilantitanbis(dimethylamid)
(t-Butylamido)(9-η⁵-fluorenyl)dimethylsilantitanbis(diethylamid)
(Phenylamido)(9-η⁵-fluorenyl)dimethylsilantitandichlorid
(Phenylamido)(9-η⁵-fluorenyl)dimethylsilantitandibromid
(Phenylamido)(9-η⁵-fluorenyl)dimethylsilantitanbis(dimethylamid)
(Phenylamido)(9-η⁵-fluorenyl)dimethylsilantitanbis(diethylamid)
(Cyclohexylamido)(9-η⁵-fluorenyl)dimethylsilantitandichlorid
(Cyclohexylamido)(9-η⁵-fluorenyl)dimethylsilantitandibromid
(Cyclohexylamido)(9-η⁵-fluorenyl)dimethylsilantitanbis(dimethylamid)
(Cyclohexylamido)(9-η⁵-fluorenyl)dimethylsilantitanbis(diethylamid)
1-(Methylamido)-2- (η5-cyclopentadienyl)ethandiylzirkondichlorid
1-(Methylamido)-2-(η5-cyclopentadienyl)ethandiylzirkonbis(diethylamid)
1-(Ethylamido)-2-(η5-cyclopentadienyl)ethandiylzirkondichlorid
1-(Ethylamido)-2-(η5-cyclopentadienyl)ethandiyldimethylsilanzirkonbis(diethylamid)
1-(Isopropylamido)-2-(η5-cyclopentadienyl)ethandiylzirkondichlorid
1-(Isopropylamido)-2-(η5-cyclopentadienyl)ethandiylzirkonbis(diethylamid)
1-1-(t-Butylamido)-2-(η5-cyclopentadienyl)ethandiylzirkondichlorid
1-1-(t-Butylamido)-2-(η5-cyclopentadienyl)ethandiylzirkonbis(diethylamid)
1-(Phenylamido)-2-(η5-cyclopentadienyl)ethandiylzirkondichlorid
1-(Phenylamido)-2-(η5-cyclopentadienyl)ethandiylzirkonbis(diethylamid)
1-(Cyclohexylamido)-2-(η5-cyclopentadienyl)ethandiylzirkondichlorid
1-(Cyclohexylamido)-2-(η5-cyclopentadienyl)ethandiylzirkonbis(diethylamid)
1-(Methylamido)-2-(η5-cyclopentadienyl)ethandiyltitandichlorid
1-(Methylamido)-2-(η5-cyclopentadienyl)ethandiyltitandibromid
1-(Methylamido)-2-(η5-cyclopentadienyl)ethandiyltitanbis(dimethylamid)
1-(Methylamido)-2-(η5-cyclopentadienyl)ethandiyltitanbis(diethylamid)
1-(Ethylamido)-2-(η5-cyclopentadienyl)ethandiyltitandichlorid
1-(Ethylamido)-2-(η⁵-cyclopentadienyl)ethandiyltitandibromid
1-(Ethylamido)-2-(η⁵-cyclopentadienyl)ethandiyltitanbis(dimethylamid)
1-(Ethylamido)-2-(η⁵-cyclopentadienyl)ethandiyltitanbis(diethylamid)
1-(Isopropylamido)-2-(η⁵-cyclopentadienyl)ethandiyltitandichlorid
1-(Isopropylamido)-2-(η⁵-cyclopentadienyl)ethandiyltitandibromid
1-(Isopropylamido)-2-(η⁵-cyclopentadienyl)ethandiyltitanbis(dimethylamid)
1-(Isopropylamido)-2-(η⁵-cyclopentadienyl)ethandiyltitanbis(diethylamid)
1-(t-Butylamido)-2-(η⁵-cyclopentadienyl)ethandiyltitandichlorid
1-(t-Butylamido)-2-(η⁵-cyclopentadienyl)ethandiyltitandibromid
1-(t-Butylamido)-2-(η⁵-cyclopentadienyl)ethandiyltitanbis(dimethylamid)
1-(t-Butylamido)-2-(η5-cyclopentadienyl)ethandiyltitanbis(diethylamid)
1-(Phenylamido)-2-(η5-cyclopentadienyl)ethandiyltitandichlorid
1-(Phenylamido)-2-(η5-cyclopentadienyl)ethandiyltitandibromid
1-(Phenylamido)-2-(η5-cyclopentadienyl)ethandiyltitanbis(dimethylamid)
1-(Phenylamido)-2-(η5-cyclopentadienyl)ethandiyltitanbis(diethylamid)
1-(Cyclohexylamido)-2-(η5-cyclopentadienyl)ethandiyltitandichlorid
1-(Cyclohexylamido)-2-(η5-cyclopentadienyl)ethandiyltitandibromid
1-(Cyclohexylamido)-2-(η5-cyclopentadienyl)ethandiyltitanbis(dimethylamid)
1-(Cyclohexylamido)-2-(η5-cyclopentadienyl)ethandiyltitanbis(diethylamid)
1-(Methylamido)-2-(3-methyl-η5-cyclopentadienyl)ethandiylzirkondichlorid
1-(Methylamido)-2-(3-methyl-η⁵-cyclopentadienyl)ethandiylzirkonbis(diethylamid)
1-(Ethylamido)-2-(3-methyl-η⁵-cyclopentadienyl)ethandiylzirkondichlorid
1-(Ethylamido)-2-(3-methyl-η⁵-cyclopentadienyl)ethandiylzirkonbis(diethylamid)
1-(Isopropylamido)-2-(3-methyl-η⁵-cyclopentadienyl)ethandiylzirkondichlorid
1-(Isopropylamido)-2-(3-methyl-η⁵-cyclopentadienyl)ethandiylzirkonbis(diethylamid)
1-(t-Butylamido)-2-(3-methyl-η⁵-cyclopentadienyl)ethandiylzirkondichlorid
1-(t-Butylamido)-2-(3-methyl-η⁵-cyclopentadienyl)ethandiylzirkonbis(diethylamid)
1-(Phenylamido)-2-(3-methyl-η⁵-cyclopentadienyl)ethandiylzirkondichlorid
1-(Phenylamido)-2-(3-methyl-η⁵-cyclopentadienyl)ethandiylzirkonbis(diethylamid)
1-(Cyclohexylamido)-2-(3-methyl-η⁵-cyclopentadienyl)ethandiylzirkondichlorid
1-(Cyclohexylamido)-2-(3-methyl-η⁵-cyclopentadienyl)ethandiylzirkonbis(diethylamid)
1-(Methylamido)-2-(3-methyl-η⁵-cyclopentadienyl)ethandiyltitandichlorid
1-(Methylamido)-2-(3-methyl-η⁵-cyclopentadienyl)ethandiyltitandibromid
1-(Methylamido)-2-(3-methyl-η⁵-cyclopentadienyl)ethandiyltitanbis(dimethylamid)
1-(Methylamido)-2-(3-methyl-η⁵-cyclopentadienyl)ethandiyltitanbis(diethylamid)
1-(Ethylamido)-2-(3-methyl-η⁵-cyclopentadienyl)ethandiyltitandichlorid
1-(Ethylamido)-2-(3-methyl-η⁵-cyclopentadienyl)ethandiyltitandibromid
1-(Ethylamido)-2-(3-methyl-η⁵-cyclopentadienyl)ethandiyltitanbis(dimethylamid)
1-(Ethylamido)-2-(3-methyl-η⁵-cyclopentadienyl)ethandiyltitanbis(diethylamid)
1-(Isopropylamido)-2-(3-methyl-η⁵-cyclopentadienyl)ethandiyltitandichlorid
1-(Isopropylamido)-2-(3-methyl-η⁵-cyclopentadienyl)ethandiyltitandibromid
1-(Isopropylamido)-2-(3-methyl-η⁵-cyclopentadienyl)ethandiyltitanbis(dimethylamid)
1-(Isopropylamido)-2-(3-methyl-η⁵-cyclopentadienyl)ethandiyltitanbis(diethylamid)
1-(t-Butylamido)-2-(3-methyl-η⁵-cyclopentadienyl)ethandiyltitandichlorid
1-(t-Butylamido)-2-(3-methyl-η⁵-cyclopentadienyl)ethandiyltitandibromid
1-(t-Butylamido)-2-(3-methyl-η⁵-cyclopentadienyl)ethandiyltitanbis(dimethylamid)
1-(t-Butylamido)-2-(3-methyl-η⁵-cyclopentadienyl)ethandiyltitanbis(diethylamid)
1-(Phenylamido)-2-(3-methyl-η⁵-cyclopentadienyl)ethandiyltitandichlorid
1-(Phenylamido)-2-(3-methyl-η⁵-cyclopentadienyl)ethandiyltitandibromid
1-(Phenylamido)-2-(3-methyl-η⁵-cyclopentadienyl)ethandiyltitanbis(dimethylamid)
1-(Phenylamido)-2-(3-methyl-η⁵-cyclopentadienyl)ethandiyltitanbis(diethylamid)
1-(Cyclohexylamido)-2-(3-methyl-η⁵-cyclopentadienyl)ethandiyltitandichlorid
1-(Cyclohexylamido)-2-(3-methyl-η⁵-cyclopentadienyl)ethandiyltitandibromid
1-(Cyclohexylamido)-2-(3-methyl-η⁵-cyclopentadienyl)ethandiyltitanbis(dimethylamid)
1-(Cyclohexylamido)-2-(3-methyl-η⁵-cyclopentadienyl)ethandiyltitanbis(diethylamid)
1-(Methylamido)(3-ethyl-η⁵-cyclopentadienyl)ethandiylzirkondichlorid
1-(Methylamido)(3-ethyl-η⁵-cyclopentadienyl)ethandiylzirkonbis(diethylamid)
1-(Ethylamido)(3-ethyl-η⁵-cyclopentadienyl)ethandiylzirkondichlorid
1-(Ethylamido)(3-ethyl-η⁵-cyclopentadienyl)ethandiylzirkonbis(diethylamid)
1-(Isopropylamido)(3-ethyl-η⁵-cyclopentadienyl)ethandiylzirkondichlorid
1-(Isopropylamido)(3-ethyl-η⁵-cyclopentadienyl)ethandiylzirkonbis(diethylamid)
1-(t-Butylamido)(3-ethyl-η⁵-cyclopentadienyl)ethandiylzirkondichlorid
1-(t-Butylamido)(3-ethyl-η⁵-cyclopentadienyl)ethandiylzirkonbis(diethylamid)
1-(Phenylamido)(3-ethyl-η⁵-cyclopentadienyl)ethandiylzirkondichlorid
1-(Phenylamido)(3-ethyl-η⁵-cyclopentadienyl)ethandiylzirkonbis(diethylamid)
1-(Cyclohexylamido)-2-(3-methyl-η⁵-cyclopentadienyl)ethandiylzirkondichlorid
1-(Cyclohexylamido)(3-ethyl-η⁵-cyclopentadienyl)ethandiylzirkonbis(diethylamid)
1-(Methylamido)(3-ethyl-η⁵-cyclopentadienyl)ethandiyltitandichlorid
1-(Methylamido)(3-ethyl-η⁵-cyclopentadienyl)ethandiyltitandibromid
1-(Methylamido)(3-ethyl-η⁵-cyclopentadienyl)ethandiyltitanbis(dimethylamid)
1-(Methylamido)(3-ethyl-η⁵-cyclopentadienyl)ethandiyltitanbis(diethylamid)
1-(Ethylamido)(3-ethyl-η⁵-cyclopentadienyl)ethandiyltitandichlorid
1-(Ethylamido)(3-ethyl-η⁵-cyclopentadienyl)ethandiyltitandibromid
1-(Ethylamido)(3-ethyl-η⁵-cyclopentadienyl)ethandiyltitanbis(dimethylamid)
1-(Ethylamido)(3-ethyl-η⁵-cyclopentadienyl)ethandiyltitanbis(diethylamid)
1-(Isopropylamido)(3-ethyl-η⁵-cyclopentadienyl)ethandiyltitandichlorid
1-(Isopropylamido)(3-ethyl-η⁵-cyclopentadienyl)ethandiyltitandibromid
1-(Isopropylamido)(3-ethyl-η⁵-cyclopentadienyl)ethandiyltitanbis(dimethylamid)
1-(Isopropylamido)(3-ethyl-η⁵-cyclopentadienyl)ethandiyltitanbis(diethylamid)
1-(t-Butylamido)(3-ethyl-η⁵-cyclopentadienyl)ethandiyltitandichlorid
1-(t-Butylamido)(3-ethyl-η⁵-cyclopentadienyl)ethandiyltitandibromid
1-(t-Butylamido)(3-ethyl-η⁵-cyclopentadienyl)ethandiyltitanbis(dimethylamid)
1-(t-Butylamido)(3-ethyl-η⁵-cyclopentadienyl)ethandiyltitanbis(diethylamid)
1-(Phenylamido)(3-ethyl-η⁵-cyclopentadienyl)ethandiyltitandichlorid
1-(Phenylamido)(3-ethyl-η⁵-cyclopentadienyl)ethandiyltitandibromid
1-(Phenylamido)(3-ethyl-η⁵-cyclopentadienyl)ethandiyltitanbis(dimethylamid)
1-(Phenylamido)(3-ethyl-η⁵-cyclopentadienyl)ethandiyltitanbis(diethylamid)
1-(Cyclohexylamido)-2-(3-methyl-η⁵-cyclopentadienyl)ethandiyltitandichlorid
1-(Cyclohexylamido)(3-ethyl-η⁵-cyclopentadienyl)ethandiyltitandibromid
1-(Cyclohexylamido)(3-ethyl-η⁵-cyclopentadienyl)ethandiyltitanbis(dimethylamid)
1-(Cyclohexylamido)(3-ethyl-η⁵-cyclopentadienyl)ethandiyltitanbis(diethylamid)
1-(Methylamido)-2-(tetramethyl-η⁵-cyclopentadienyl)ethandiylzirkondichlorid
1-(Methylamido)-2-(tetramethyl-η⁵-cyclopentadienyl)ethandiylzirkonbis(diethylamid)
1-(Ethylamido)-2-(tetramethyl-η⁵-cyclopentadienyl)ethandiylzirkondichlorid
1-(Ethylamido)-2-(tetramethyl-η⁵-cyclopentadienyl)ethandiylzirkonbis(diethylamid)
1-(Isopropylamido)-2-(tetramethyl-η⁵-cyclopentadienyl)ethandiylzirkondichlorid
1-(Isopropylamido)-2-(tetramethyl-η⁵-cyclopentadienyl)ethandiylzirkonbis(diethylamid)
1-(t-Butylamido)-2-(tetramethyl-η⁵-cyclopentadienyl)ethandiylzirkondichlorid
1-(t-Butylamido)-2-(tetramethyl-η⁵-cyclopentadienyl)ethandiylzirkonbis(diethylamid)
1-(Phenylamido)-2-(tetramethyl-η⁵-cyclopentadienyl)ethandiylzirkondichlorid
1-(Phenylamido)-2-(tetramethyl-η⁵-cyclopentadienyl)ethandiylzirkonbis(diethylamid)
1-(Cyclohexylamido)-2-(tetramethyl-η⁵-cyclopentadienyl)ethandiylzirkondichlorid
1-(Cyclohexylamido)-2-(tetramethyl-η⁵-cyclopentadienyl)ethandiylzirkonbis(diethylamid)
1-(Methylamido)-2-(tetramethyl-η⁵-cyclopentadienyl)ethandiyltitandichlorid
1-(Methylamido)-2-(tetramethyl-η⁵-cyclopentadienyl)ethandiyltitandibromid
1-(Methylamido)-2-(tetramethyl-η⁵-cyclopentadienyl)ethandiyltitanbis(dimethylamid)
1-(Methylamido)-2-(tetramethyl-η⁵-cyclopentadienyl)ethandiyltitanbis(diethylamid)
1-(Ethylamido)-2-(tetramethyl-η⁵-cyclopentadienyl)ethandiyltitandichlorid
1-(Ethylamido)-2-(tetramethyl-η⁵-cyclopentadienyl)ethandiyltitandibromid
1-(Ethylamido)-2-(tetramethyl-η⁵-cyclopentadienyl)ethandiyltitanbis(dimethylamid)
1-(Ethylamido)-2-(tetramethyl-η⁵-cyclopentadienyl)ethandiyltitanbis(diethylamid)
1-(Isopropylamido)-2-(tetramethyl-η⁵-cyclopentadienyl)ethandiyltitandichlorid
1-(Isopropylamido)-2-(tetramethyl-η⁵-cyclopentadienyl)ethandiyltitandibromid
1-(Isopropylamido)-2-(tetramethyl-η⁵-cyclopentadienyl)ethandiyltitanbis(dimethylamid)
1-(Isopropylamido)-2-(tetramethyl-η⁵-cyclopentadienyl)ethandiyltitanbis(diethylamid)
1-(t-Butylamido)-2-(tetramethyl-η⁵-cyclopentadienyl)ethandiyltitandichlorid
1-(t-Butylamido)-2-(tetramethyl-η⁵-cyclopentadienyl)ethandiyltitandibromid
1-(t-Butylamido)-2-(tetramethyl-η⁵-cyclopentadienyl)ethandiyltitanbis(dimethylamid)
1-(t-Butylamido)-2-(tetramethyl-η⁵-cyclopentadienyl)ethandiyltitanbis(diethylamid)
1-(Phenylamido)-2-(tetramethyl-η⁵-cyclopentadienyl)ethandiyltitandichlorid
1-(Phenylamido)-2-(tetramethyl-η⁵-cyclopentadienyl)ethandiyltitandibromid
1-(Phenylamido)-2-(tetramethyl-η⁵-cyclopentadienyl)ethandiyltitanbis(dimethylamid)
1-(Phenylamido)-2-(tetramethyl-η⁵-cyclopentadienyl)ethandiyltitanbis(diethylamid)
1-(Cyclohexylamido)-2-(tetramethyl-η⁵-cyclopentadienyl)ethandiyltitandichlorid
1-(Cyclohexylamido)-2-(tetramethyl-η⁵-cyclopentadienyl)ethandiyltitandibromid
1-(Cyclohexylamido)-2-(tetramethyl-η⁵-cyclopentadienyl)ethandiyltitanbis(dimethylamid)
1-(Cyclohexylamido)-2-(tetramethyl-η⁵-cyclopentadienyl)ethandiyltitanbis(diethylamid)
1-(Methylamido)-2-(1-η⁵-indenyl)ethandiylzirkondichlorid
1-(Methylamido)-2-(1-η⁵-indenyl)ethandiylzirkonbis(diethylamid)
1-(Ethylamido)-2-(1-η⁵-indenyl)ethandiylzirkondichlorid
1-(Ethylamido)-2-(1-η⁵-indenyl)ethandiylzirkonbis(diethylamid)
1-(Isopropylamido)-2-(1-η⁵-indenyl)ethandiylzirkondichlorid
1-(Isopropylamido)-2-(1-η⁵-indenyl)ethandiylzirkonbis(diethylamid)
1-(t-Butylamido)-2-(1-η⁵-indenyl)ethandiylzirkondichlorid
1-(t-Butylamido)-2-(1-η⁵-indenyl)ethandiylzirkonbis(diethylamid)
1-(Phenylamido)-2-(1-η⁵-indenyl)ethandiylzirkondichlorid
1-(Phenylamido)-2-(1-η⁵-indenyl)ethandiylzirkonbis(diethylamid)
1-(Cyclohexylamido)-2-(1-η⁵-indenyl)ethandiylzirkondichlorid
1-(Cyclohexylamido)-2-(1-η⁵-indenyl)ethandiylzirkonbis(diethylamid)
1 -(Methylamido)-2-(1-η⁵-indenyl)ethandiyltitandichlorid
1-(Methylamido)-2-(1-η⁵-indenyl)ethandiyltitandibromid
1-(Methylamido)-2-(1-η⁵-indenyl)ethandiyltitanbis(dimethylamid)
1-(Methylamido)-2-(1-η⁵-indenyl)ethandiyltitanbis(diethylamid)
1-(Ethylamido)-2-(1-η⁵-indenyl)ethandiyltitandichlorid
1-(Ethylamido)-2-(1-η⁵-indenyl)ethandiyltitandibromid
1-(Ethylamido)-2-(1-η⁵-indenyl)ethandiyltitanbis(dimethylamid)
1-(Ethylamido)-2-(1-η⁵-indenyl)ethandiyltitanbis(diethylamid)
1-(Isopropylamido)-2-(1-η⁵-indenyl)ethandiyltitandichlorid
1-(Isopropylamido)-2-(1-η⁵-indenyl)ethandiyltitandibromid
1-(Isopropylamido)-2-(1-η⁵-indenyl)ethandiyltitanbis(dimethylamid)
1-(Isopropylamido)-2-(1-η⁵-indenyl)ethandiyltitanbis(diethylamid)
1-(t-Butylamido)-2-(1-η⁵-indenyl)ethandiyltitandichlorid
1 -(t-Butylamido)-2-(1-η⁵-indenyl)ethandiyltitandibromid
1-(t-Butylamido)-2-(1-η⁵-indenyl)ethandiyltitanbis(dimethylamid)
1-(t-Butylamido)-2-(1-η⁵-indenyl)ethandiyltitanbis(diethylamid)
1-(Phenylamido)-2-(1-η⁵-indenyl)ethandiyltitandichlorid
1-(Phenylamido)-2-(1-η⁵-indenyl)ethandiyltitandibromid
1-(Phenylamido)-2-(1-η⁵-indenyl)ethandiyltitanbis(dimethylamid)
1-(Phenylamido)-2-(1-η⁵-indenyl)ethandiyltitanbis(diethylamid)
1 -(Cyclohexylamido)-2-(1-η⁵-indenyl)ethandiyltitandichlorid
1-(Cyclohexylamido)-2-(1-η⁵-indenyl)ethandiyltitandibromid
1-(Cyclohexylamido)-2-(1-η⁵-indenyl)ethandiyltitanbis(dimethylamid)
1-(Cyclohexylamido)-2-(1-η⁵-indenyl)ethandiyltitanbis(diethylamid)
1-(Methylamido)-2-(9-η⁵-fluorenyl)ethandiylzirkondichlorid
1-(Methylamido)-2-(9-η⁵-fluorenyl)ethandiylzirkonbis(diethylamid)
1-(Ethylamido)-2-(9-η⁵-fluorenyl)ethandiylzirkondichlorid
1-(Ethylamido)-2-(9-η⁵-fluorenyl)ethandiylzirkonbis(diethylamid)
1-(Isopropylamido)-2-(9-η⁵-fluorenyl)ethandiylzirkondichlorid
1-(Isopropylamido)-2-(9-η⁵-fluorenyl)ethandiylzirkonbis(diethylamid)
1-(t-Butylamido)-2-(9-η⁵-fluorenyl)ethandiylzirkondichlorid
1-(t-Butylamido)-2-(9-η⁵-fluorenyl)ethandiylzirkonbis(diethylamid)
1-(Phenylamido)-2-(9-η⁵-fluorenyl)ethandiylzirkondichlorid
1-(Phenylamido)-2-(9-η⁵-fluorenyl)ethandiylzirkonbis(diethylamid)
1-(Cyclohexylamido)-2-(9-η⁵-fluorenyl)ethandiylzirkondichlorid
1-(Cyclohexylamido)-2-(9-η⁵-fluorenyl)ethandiylzirkonbis(diethylamid)
1-(Methylamido)-2-(9-η⁵-fluorenyl)ethandiyltitandichlorid
1-(Methylamido)-2-(9-η⁵-fluorenyl)ethandiyltitandibromid
1-(Methylamido)-2-(9-η⁵-fluorenyl)ethandiyltitanbis(dimethylamid)
1-(Methylamido)-2-(9-η⁵-fluorenyl)ethandiyltitanbis(diethylamid)
1-(Ethylamido)-2-(9-η⁵-fluorenyl)ethandiyltitandichlorid
1-(Ethylamido)-2-(9-η⁵-fluorenyl)ethandiyltitandibromid
1-(Ethylamido)-2-(9-η⁵-fluorenyl)ethandiyltitanbis(dimethylamid)
1-(Ethylamido)-2-(9-η⁵-fluorenyl)ethandiyltitanbis(diethylamid)
1-(Isopropylamido)-2-(9-η⁵-fluorenyl)ethandiyltitandichlorid
1-(Isopropylamido)-2-(9-η⁵-fluorenyl)ethandiyltitandibromid
1-(Isopropylamido)-2-(9-η⁵-fluorenyl)ethandiyltitanbis(dimethylamid)
1-(Isopropylamido)-2-(9-η⁵-fluorenyl)ethandiyltitanbis(diethylamid)
1-(t-Butylamido)-2-(9-η⁵-fluorenyl)ethandiyltitandichlorid
1-(t-Butylamido)-2-(9-η⁵-fluorenyl)ethandiyltitandibromid
1-(t-Butylamido)-2-(9-η⁵-fluorenyl)ethandiyltitanbis(dimethylamid)
1-(t-Butylamido)-2-(9-η⁵-fluorenyl)ethandiyltitanbis(diethylamid)
1-(Phenylamido)-2-(9-η⁵-fluorenyl)ethandiyltitandichlorid
1-(Phenylamido)-2-(9-η⁵-fluorenyl)ethandiyltitandibromid
1-(Phenylamido)-2-(9-η⁵-fluorenyl)ethandiyltitanbis(dimethylamid)
1-(Phenylamido)-2-(9-η⁵-fluorenyl)ethandiyltitanbis(diethylamid)
1-(Cyclohexylamido)-2-(9-η⁵-fluorenyl)ethandiyltitandichlorid
1-(Cyclohexylamido)-2-(9-η⁵-fluorenyl)ethandiyltitandibromid
1-(Cyclohexylamido)-2-(9-η⁵-fluorenyl)ethandiyltitanbis(dimethylamid)
1-(Cyclohexylamido)-2-(9-η⁵-fluorenyl)ethandiyltitanbis(diethylamid)

Der für das erfindungsgemäße Verfahren zu verwendende Metall-Koordinationskomplex kann auch in dimerer Form vorliegen. Beispiele hierfür sind:
Bis[(isopropylamido)(η⁵-cyclopentadienyl)dimethylsilan]zirkonium
Bis[(t-butylamido)(η⁵-cyclopentadienyl)dimethylsilan]zirkonium
Bis[(phenylamido)(η⁵-cyclopentadienyl)dimethylsilan]zirkonium
Bis[(cyclohexylamido)(_η⁵-cyclopentadienyl)dimethylsilan]zirkonium
Bis[(isopropylamido)(3-methyl-η⁵-cyclopentadienyl)dimethylsilan]zirkonium
Bis[(t-butylamido)(3-methyl-η⁵-cyclopentadienyl)dimethylsilan]zirkonium
Bis[(phenylamido)(3-methyl-η⁵-cyclopentadienyl)dimethylsilan]zirkonium
Bis[(cyclohexylamido)(3-methyl-η⁵-cyclopentadienyl)dimethylsilan]zirkonium
Bis[(isopropylamido)(tetramethyl-η⁵-cyclopentadienyl)dimethylsilan]zirkonium
Bis[(t-butylamido)(tetramethyl-η⁵-cyclopentadienyl)dimethylsilan]zirkonium
Bis[(phenylamido)(tetramethyl-η⁵-cyclopentadienyl)dimethylsilan]zirkonium
Bis[(cyclohexylamido)(tetramethyl-η⁵-cyclopentadienyl)dimethylsilan]zirkonium
Bis[(isopropylamido)(1-η⁵-indenyl)dimethylsilan]zirkonium
Bis[(t-butylamido)(1-η⁵-indenyl)dimethylsilan)zirkonium
Bis[(phenylamido)(1-η⁵-indenyl)dimethylsilan]zirkonium
Bis[(cyclohexylamido)(1-η⁵-indenyl)dimethylsilan]zirkonium
Bis[(isopropylamido)(9-η⁵-fluorenyl)dimethylsilan]zirkonium
Bis[(t-butylamido)(9-η⁵-fluorenyl)dimethylsilan]zirkonium
Bis[(phenylamido)(9-η⁵-fluorenyl)dimethylsilan]zirkonium
Bis[(cyclohexylamido)(9-η⁵-fluorenyl)dimethylsilan]zirkonium
Bis[(isopropylamido)(η⁵-cyclopentadienyl)dimethylsilan]titan
Bis[(t-butylamido)(η⁵-cyclopentadienyl)dimethylsilan]titan
Bis[(phenylamido)(η⁵-cyclopentadienyl)dimethylsilan]titan
Bis[(cyclohexylamido)(_η⁵-cyclopentadienyl)dimethylsilan]titan
Bis[(isopropylamido)(3-methyl-η⁵-cyclopentadienyl)dimethylsilan]titan
Bis[(t-butylamido)(3-methyl-η⁵-cyclopentadienyl)dimethylsilan]titan
Bis[(phenylamido)(3-methyl-η⁵-cyclopentadienyl)dimethylsilan]titan
Bis[(cyclohexylamido)(3-methyl-η⁵-cyclopentadienyl)dimethylsilan]titan
Bis[(isopropylamido)(tetramethyl-η⁵-cyclopentadienyl)dimethylsilan]titan
Bis[(t-butylamido)(tetramethyl-η⁵-cyclopentadienyl)dimethylsilan]titan
Bis[(phenylamido)(tetramethyl-η⁵-cyclopentadienyl)dimethylsilan]titan
Bis[(cyclohexylamido)(tetramethyl-η⁵-cyclopentadienyl)dimethylsilan]titan
Bis[(isopropylamido)(1-η⁵-indenyl)dimethylsilan]titan
Bis[(t-butylamido)(1-η⁵-indenyl)dimethylsilan]titan
Bis[(phenylamido)(1-η⁵-indenyl)dimethylsilan]titan
Bis[(cyclohexylamido)(1-η⁵-indenyl)dimethylsilan)titan
Bis[(isopropylamido)(9-η⁵-fluorenyl)dimethylsilan]titan
Bis[(t-butylamido)(9-η⁵-fluorenyl)dimethylsilan]titan
Bis[(phenylamido)(9-η⁵-fluorenyl)dimethylsilan]titan
Bis[(cyclohexylamido)(9-η⁵-fluorenyl)dimethylsilan]titan
Bis[(isopropylamido)(η⁵-cyclopentadienyl)dimethylsilan]hafnium
Bis[(t-butylamido)(η⁵-cyclopentadienyl)dimethylsilan]hafnium
Bis[(phenylamido)(η⁵-cyclopentadienyl)dimethylsilan]hafnium
Bis[(cyclohexylamido)(_η⁵-cyclopentadienyl)dimethylsilan]hafnium
Bis[(isopropylamido)(3-methyl-η⁵-cyclopentadienyl)dimethylsilan]hafnium
Bis[(t-butylamido)(3-methyl-η⁵-cyclopentadienyl)dimethylsilan]hafnium
Bis[(phenylamido)(3-methyl-η⁵-cyclopentadienyl)dimethylsilan]hafnium
Bis[(cyclohexylamido)(3-methyl-η⁵-cyclopentadienyl)dimethylsilan]hafnium
Bis[(isopropylamido)(tetramethyl-η⁵-cyclopentadienyl)dimethylsilan]hafnium
Bis[(t-butylamido)(tetramethyl-η⁵-cyclopentadienyl)dimethylsilan]hafnium
Bis[(phenylamido)(tetramethyl-η⁵-cyclopentadienyl)dimethylsilan]hafnium
Bis[(cyclohexylamido)(tetramethyl-η⁵-cyclopentadienyl)dimethylsilan]hafnium
Bis[(isopropylamido)(1-η⁵-indenyl)dimethylsilan]hafnium
Bis[(t-butylamido)(1-η⁵-indenyl)dimethylsilan]hafnium
Bis[(phenylamido)(1-η⁵-indenyl)dimethylsilan]hafnium
Bis[(cyclohexylamido)(1-η⁵-indenyl)dimethylsilan]hafnium
Bis[(isopropylamido)(9-η⁵-fluorenyl)dimethylsilan]hafnium
Bis[(t-butylamido)(9-η⁵-fluorenyl)dimethylsilan]hafnium
Bis[(phenylamido)(9-η⁵-fluorenyl)dimethylsilan]hafnium
Bis[(cyclohexylamido)(9-η⁵-fluorenyl)dimethylsilan]hafnium

Die Herstellung der für das erfindungsgemäße Verfahren zu verwendenden Metall-Koordinations-Komplexe ist in EP-A-426 815 ausführlich beschrieben.

In dem erfindungsgemäßen Verfahren wird als Cokatalysator bevorzugt ein Aluminoxan eingesetzt, welches vorzugsweise die Formel IIa für den linearen Typ und/oder die Formel IIb für den cyclischen Typ aufweist, 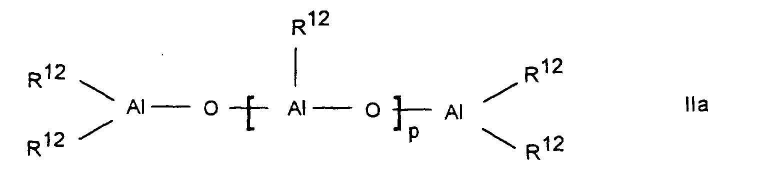 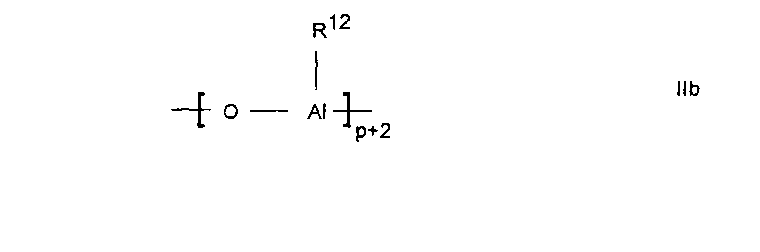 wobei in den Formeln IIa und IIb die Reste R¹² gleich oder verschieden sind und eine C₁-C₆-Alkylgruppe, eine C₆-C₁₈-Arylgruppe, Benzyl oder Wasserstoff bedeuten, und p eine ganze Zahl von 2 bis 50, bevorzugt 10 bis 35 bedeutet. Bevorzugt sind die Reste R¹² gleich und bedeuten Methyl, Isobutyl, Phenyl oder Benzyl, besonders bevorzugt Methyl.

Sind die Reste R¹² verschieden, so sind sie bevorzugt Methyl und Wasserstoff oder alternativ Methyl und isobutyl, wobei Wasserstoff oder isobutyl bevorzugt mit einen zahlenmäßigen Anteil von 0,01 bis 40% (der Reste R¹²) enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder eine Hydridoaluminiumkohlenwasserstoffverbindung mit Wasser (gasförmig, fest, flüssig oder gebunden, beispielsweise als Kristallwasser) in einem inerten Lösungsmittel (wie Toluol) umgesetzt wird. Zur Herstellung eines Aluminoxans mit verschiedenen Alkylgruppen R¹² werden entsprechend der gewünschten Zusammensetzung zwei verschiedene Aluminiumtrialkyle (AlR₃ + AlR'₃) mit Wasser umgesetzt (S. Pasynkiewicz, Polyhedron 9 (1990) 429, EP-A-302 424). Die genaue räumliche Struktur der Aluminoxane ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist auch möglich, das Aluminoxan auf einen Träger aufzubringen und es dann als Suspension in geträgerter Form einzusetzen. Es sind mehrere Trägerungsverfahren bekannt (EP-A-578 838). Kieselgel kann als Träger fungieren.

Der für das erfindungsgemäße Verfahren zu verwendende Metall-Koordinations-Komplex wird vor dem Einsatz in der Polymerisationsreaktion mit einem Cokatalysator, insbesondere einem Aluminoxan voraktiviert. Dadurch wird die Polymerisationsaktivität deutlich erhöht.

Die Voraktivierung der Übergangsmetallverbindung wird in Lösung vorgenommen. Bevorzugt wird dabei der Metall-Koordinations-Komplex in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Der Metall-Koordinations-Komplex kann in der gleichen Konzentration eingesetzt werden, vorzugsweise wird es jedoch in einer Menge von 10⁻⁴ bis 1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78 bis 100 °C, vorzugsweise 0 bis 70 °C.

Mit Hilfe des Metall-Koordinations-Komplexes kann eine Vorpolymerisation erfolgen. Zur Vorpolymerisation wird bevorzugt das (oder eines der) in der Polymerisation eingesetzte(n) Olefin(e) verwendet.

Der Metall-Koordinations-Komplex kann auch auf einen Träger aufgebracht werden. Geeignete Träger sind beispielsweise Silikagele, Aluminiumoxide, festes Aluminoxan oder andere anorganische Trägermaterialien. Ein geeignetes Trägermaterial ist auch ein Polyolefinpulver in feinverteilter Form.

Eine weitere mögliche Ausgestaltung des erfindungsgemäßen Verfahrens besteht darin, daß man an Stelle oder neben eines Aluminoxans eine salzartige Verbindung der Formel RₓNH₄₋ₓBR'₄ oder der Formel R₃PHBR'₄ als Cokatalysator verwendet. Dabei sind x = 1, 2 oder 3, R = Alkyl oder Aryl, gleich oder verschieden, und R' = Aryl, das auch fluoriert oder teilfluoriert sein kann. In diesem Fall besteht der Katalysator aus dem Reaktionsprodukt eines Metall-Koordinations-Komplexes mit einer der genannten Verbindungen (EP-A-277 004).

Falls dem Reaktionsgemisch Lösemittel zugesetzt wird, dann handelt es sich um gebräuchliche inerte Lösemittel wie aliphatische oder cycloaliphatische Kohlenwasserstoffe, Benzin- bzw. hydrierte Dieselölfraktionen oder Toluol.

Der Metall-Koordinations-Komplex wird bevorzugt in einer Konzentration, bezogen auf das Übergangsmetall, von 10⁻¹ bis 10⁻⁸ mol, vorzugsweise 10⁻² bis 10⁻⁷ mol, besonders bevorzugt von 10⁻³ bis 10⁻⁷ mol Übergangsmetall pro dm³ Reaktorvolumen angewendet. Das Aluminoxan wird in einer Konzentration von 10⁻⁴ bis 10⁻¹, vorzugsweise 10⁻⁴ bis 2*10⁻² mol pro dm³ Reaktorvolumen verwendet, bezogen auf den Gehalt an Aluminium. Prinzipiell sind aber auch höhere Konzentrationen möglich.

Die Erfindung betrifft ein Verfahren zur Herstellung eines Cycloolefincopolymeren durch Polymerisation von 0,1 bis 99,9 Gew.-%, bezogen auf die Gesamtmenge der Monomeren, mindestens eines polycyclischen Olefins der Formel III, IV, IV', V, VI, VII oder VIII 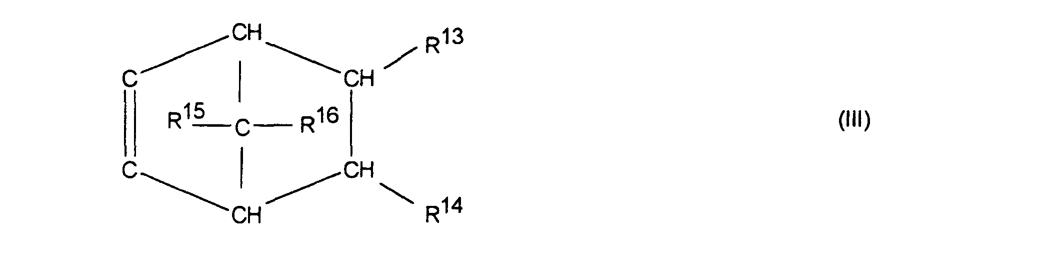 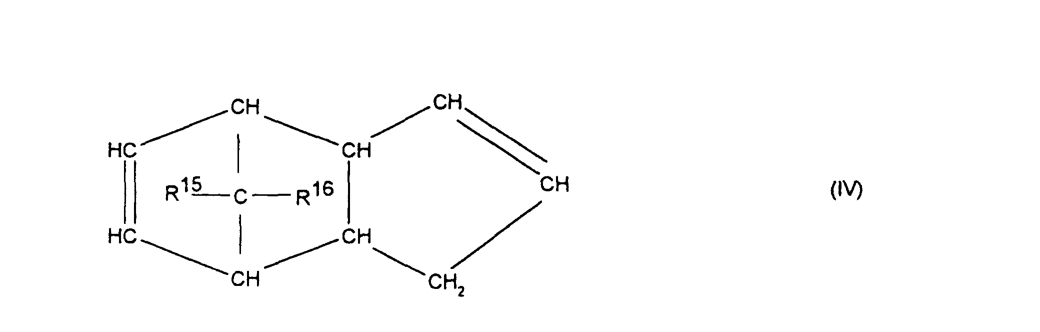 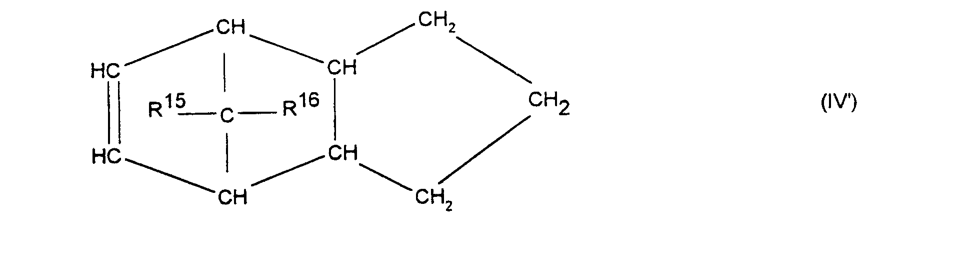 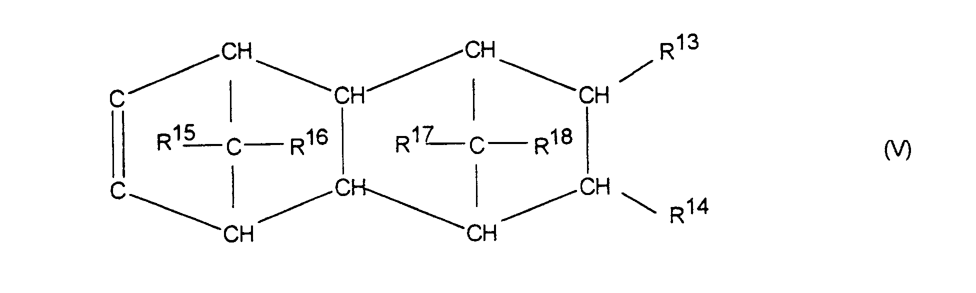 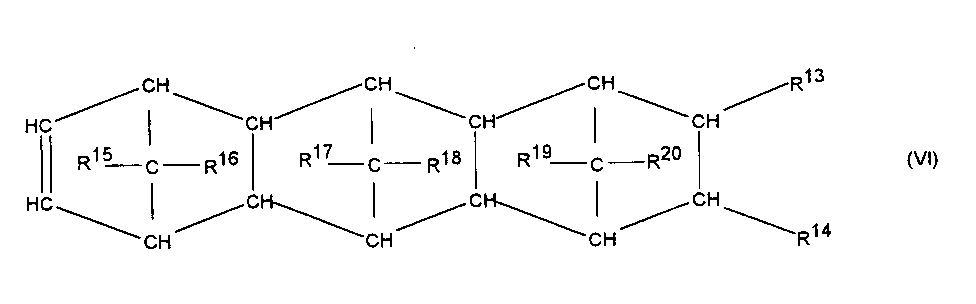 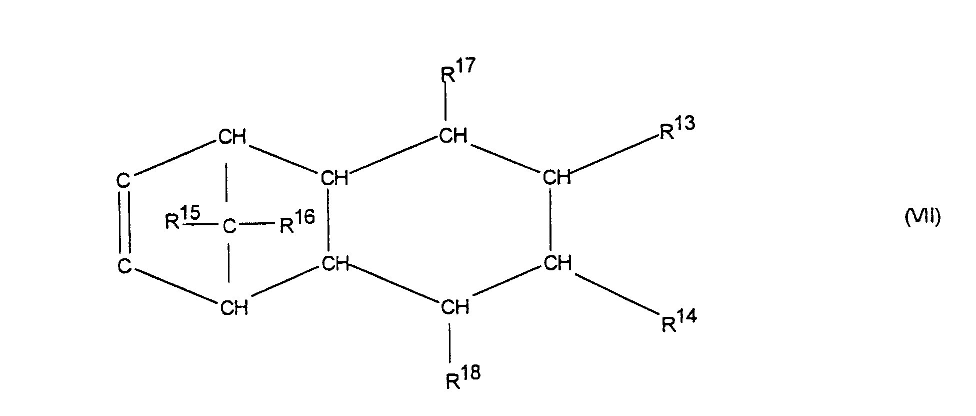 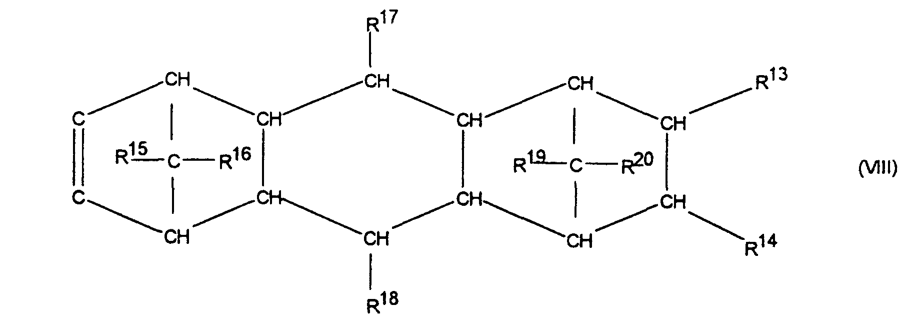 worin R¹³, R¹⁴, R¹⁵, R¹⁶, R¹⁷, R¹⁸, R¹⁹ und R²⁰ gleich oder verschieden sind und ein Wasserstoffatom oder einen Kohlenwasserstoffrest bedeuten, wobei gleiche Reste in den verschiedenen Formeln eine unterschiedliche Bedeutung haben können, 0 bis 99,9 Gew.-%, bezogen auf die Gesamtmenge der Monomeren, mindestens eines monocyclischen Olefins der Formel IX 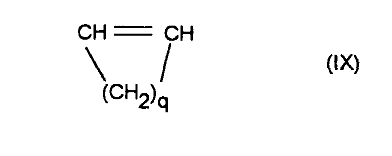 worin q eine Zahl von 2 bis 10 ist, und 0,1 bis 99,9 Gew.-%, bezogen auf die Gesamtmenge der Monomeren, mindestens eines acyclischen 1-Olefins der Formel X 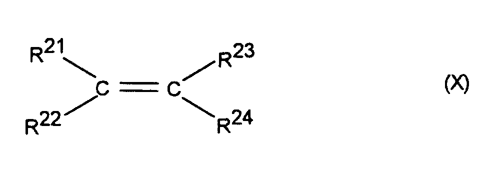 worin R²¹, R²², R²³ und R²⁴ gleich oder verschieden sind und ein Wasserstoffatom oder einen Kohlenwasserstoffrest, bevorzugt einen C₆-C₁₀-Arylrest oder einen C₁-C₈-Alkylrest bedeuten, bei Temperaturen von -78 bis 150 °C, insbesondere 0 bis 100 °C, und einem Druck von 0,01 bis 64 bar durchgeführt.

Bevorzugt sind Cycloolefine der Formeln III oder V, worin R¹³, R¹⁴, R¹⁵, R¹⁶, R¹⁷, R¹⁸, R¹⁹ und R²⁰ gleich oder verschieden sind und ein Wasserstoffatom oder einen Kohlenwasserstoffrest, insbesondere einen C₆-C₁₀-Arylrest oder einen C₁-C₈-Alkylrest bedeuten, wobei gleiche Reste in den verschiedenen Formeln eine unterschiedliche Bedeutung haben können.

Gegebenenfalls werden ein oder mehrere monocyclische Olefine der Formel IX für die Polymerisation verwendet.

Bevorzugt ist weiterhin ein acyclisches Olefin der Formel X, worin R²¹, R²², R²³ und R²⁴ gleich oder verschieden sind und ein Wasserstoffatom oder einen Kohlenwasserstoffrest, bevorzugt einen C₆-C₁₀-Arylrest oder einen C₁-C₈-Alkylrest bedeuten, wie Ethylen und Propylen.

Insbesondere werden Copolymere von polycyclischen Olefinen, vorzugsweise der Formeln III und V, mit Ethylen hergestellt.

Besonders bevorzugte polycyclische Olefine sind Norbornen und Tetracyclododecen, wobei diese durch C₁-C₆-Alkyl substituiert sein können. Sie werden vorzugsweise mit Ethylen copolymerisiert. Ganz besonders bevorzugt sind Ethylen/ Norbornen-Copolymere und Ethylen/ Tetracyclododecen-Copolymere.

Das polycyclische Olefin wird in einer Menge von 0,1 bis 99,9 Gew.-% und das monocyclische Olefin in einer Menge von 0 bis 99,9 Gew.-%, jeweils bezogen auf die Gesamtmenge der Monomeren, eingesetzt.

Die Konzentration des eingesetzten acyclischen Olefins ergibt sich aus dessen Löslichkeit in dem Reaktionsmedium bei gegebenem Druck und gegebener Temperatur.

Als polycyclische Olefine, monocyclische Olefine und acyclische Olefine sind auch Gemische zweier oder mehrerer Olefine des jeweiligen Typs zu verstehen. Das heißt, es können neben polycyclischen Bicopolymeren auch Ter- und Multicopolymere nach dem erfindungsgemäßen Verfahren hergestellt werden. Auch Copolymere monocyclischer Olefine und acyclischer Olefine können nach dem beschriebenen Verfahren erhalten werden.

Von den monocyclischen Olefinen ist Cyclopenten, das substituiert sein kann, bevorzugt.

Aufgrund ihrer geringeren Oxidations- und Vernetzungsanfälligkeit sind Cycloolefincopolymere bevorzugt, die aus Einheiten bestehen, die abgeleitet sind von Monomeren, die neben der für die Polymerisation erforderlichen olefinischen Doppelbindung keine weiteren ungesättigten Funktionalitäten aufweisen.

Bevorzugt wird das erfindungsgemäße Verfahren bei Temperaturen von -78 bis 150 °C, insbesondere 0 bis 100 °C, und einem Druck von 0,01 bis 64 bar durchgeführt.

Das erfindungsgemäße Verfahren betrifft die Herstellung von Cycloolefincopolymeren, die einen Anteil von mindestens 5 mol% des cyclischen Olefins enthalten.

Bei der Herstellung von Copolymerisaten kann die Variation der Molverhältnisse des polycyclischen Olefins zum eingesetzten offenkettigen Olefin in einem weiten Bereich erfolgen. Bevorzugt werden molare Verhältnisse von 3:1 bis 200:1 Cycloolefin zu offenkettigem Olefin eingesetzt. Durch die Wahl der Polymerisationstemperatur, durch die Konzentration der Katalysatorkomponenten und das eingesetzte Molverhältnis bzw. den Druck des gasförmigen, offenkettigen Olefins läßt sich die Einbaurate an Comonomer beinahe beliebig steuern. Bevorzugt werden Einbauraten zwischen 5 und 80 Mol-% der cyclischen Komponenten, besonders bevorzugt werden Einbauraten zwischen 10 und 60 Mol-% der cyclischen Komponenten, ganz besonders bevorzugt werden Einbauraten zwischen 15 und 40 Mol-% der cyclischen Komponenten.

Die nach dem erfindungsgemäßen Verfahren hergestellten Cycloolefincopolymere weisen Glastemperaturen zwischen -50 und 220 °C auf. Bevorzugt sind Glastemperaturen zwischen -30 und 180 °C, besonders bevorzugt sind Glastemperaturen zwischen -20 und 150 °C, ganz besonders bevorzugt sind Glastemperaturen zwischen 0 und 100 °C.

Die Polymerisation kann auch mehrstufig erfolgen, wobei auch Blockcopolymere entstehen können (DE-A-42 05 416).

Die mittlere Molmasse des gebildeten Polymers läßt sich durch Wasserstoff-Dosierung, Variation der Katalysatorkonzentration oder Variation der Temperatur in bekannter Weise steuern.

Die nach dem erfindungsgemäßen Verfahren hergestellten Cycloolefincopolymere weisen massenmittlere Molmassen M_{w} zwischen 1.000 und 10.000.000 g/mol auf. Bevorzugt sind massenmittlere Molmassen zwischen 10.000 und 5.000.000 g/mol, besonders bevorzugt sind massenmittlere Molmassen zwischen 50.000 und 2.000.000 g/mol.

Die nach dem erfindungsgemäßen Verfahren hergestellten Cycloolefincopolymere weisen Viskositätszahlen zwischen 10 und 1000 ml/g auf. Bevorzugt sind Viskositätszahlen zwischen 30 und 500 ml/g, besonders bevorzugt sind Viskositätszahlen zwischen 50 und 300 ml/g.

Bei Cycloolefincopolymeren, die nicht nach dem erfindungsgemäßen Verfahren hergestellt wurden, führt die geringe Molmasse des Cycloolefincopolymeren zu schlechten Materialeigenschaften wie geringer Zähigkeit, so daß diese Materialien für den kommerziellen Einsatz von geringem Interesse sind.

Es wurde nun überraschend festgestellt, daß nach dem erfindungsgemäßen Verfahren über einen breiten Bereich der Glastemperaturen Cycloolefincopolymere mit deutlich höheren Molmassen hergestellt werden können. Die nach dem erfindungsgemäßen Verfahren hergestellten Cycloolefincopolymere weisen eine höhere Schmelzefestigkeit und Zähigkeit auf und sind daher von besonderem Interesse für den kommerziellen Einsatz.

Die Polydispersität M_{w}/Mₙ der Copolymeren ist mit Werten von 1,6 bis 3,5 recht eng. Dadurch resultiert ein Eigenschaftsbild, das diese für das Spritzgießen besonders geeignet macht. Eine Anpassung der Polydispersität auch über die angegebenen Grenzen hinaus ist durch die Wahl des Katalysatorsystems möglich. Neben monomodalen Verteilungen sind nach dem erfindungsgemäßen Verfahren auch Cycloolefincopolymere mit bi- oder multimodalen Verteilungen herstellbar.

Bei der Wahl von Katalysatorsystemen nicht im Sinne des erfindungsgemäßen Verfahrens können neben den Cycloolefincopolymeren Ethylenpolymerisate entstehen, die die Tranparenz des Materials herabsetzen. Durch die Unlöslichkeit dieser Ethylenpolymerisate kommt es außerdem im Prozeß zu Ablagerungen, die den Produktionsablauf stören und regelmäßige Reinigungsarbeiten erfordern.

Es hat sich nun überraschenderweise gezeigt, daß mit dem für das erfindungsgemäße Verfahren zu verwendenden Katalysatorsystem keine Ethylenpolymerisate entstanden sind. Mit dem erfindungsgemäßen Verfahren lassen sich Cycloolefincopolymere hoher Transparenz herstellen.

Das erfindungsgemäße Verfahren zeichnet sich aus durch eine hohe Ausbeute an Polymer bezogen auf die Menge eingesetzten Katalysators. Das erfindungsgemäße Verfahren stellt damit ein gegenüber dem Stand der Technik sehr wirtschaftliches Verfahren dar, da die Katalysatorkosten aufgrund der hohen Aktivität des Katalysators verhältnismäßig geringer sind.

Sowohl beim Extrudieren als auch beim Spritzgießen wurden bei Temperaturen von 300 °C weder Zersetzungsreaktionen noch ein Viskositätsabbau gefunden.

Die erfindungsgemäß hergestellten Materialien eignen sich besonders zur Herstellung von Formkörpern wie Extrusionsteilen (Folien, Platten, Schläuchen, Rohren, Stangen und Fasern) oder Spritzgußartikeln beliebiger Form und Größe. Eine wichtige Eigenschaft der erfindungsgemäßen Materialien sind ihre Transparenz, ihre Reinheit, die günstigen mechanischen Eigenschaften, die geringe Wasseraufnahme und die hohe Wasserdampfsperrwirkung.

Der mit einem Abbe-Refraktometer und Mischlicht bestimmte Brechungsindex der in den nachfolgenden Beispielen beschriebenen Reaktionsprodukte liegt im Bereich zwischen 1,520 und 1,555. Nachdem der Brechungsindex sehr nahe an dem von Kronglas (n = 1,51) liegt, können die erfindungsgemäßen Produkte als Glasersatz verschiedene Anwendungen im optischen Bereich finden wie beispielsweise Linsen, Prismen, Trägerplatten und -folien für optische Datenspeicher, für Videoplatten, für Compact Disks, als Deck- und Fokussierscheiben für Solarzellen, als Deck- und Streuscheiben für Leistungsoptiken, als Lichtwellenleiter in der Form von Fasern oder Folien. Aufgrund des beschriebenen Eigenschaftsprofils sind die erfindungsgemäß hergestellten Materialien von großem Interesse für den Bereich der Medizintechnik. Sie finden Einsatz als Materialien für Katheder, Beutel für Infusionslösungen oder Dialyseflüssigkeit, für Schläuche, Behälter, Implantate, Bestandteile von medizinischen Geräten. Außerdem werden sie in Form von Spritzgußteilen auch für Behälter, Fläschchen, Gläschen, Spritzen für die Aufbewahrung, den Austausch oder die Applikation von Flüssigkeiten verwendet. Die Eigenschaften der erfindungsgemäß hergestellten Cycloolefincopolymere machen sie besonders geeignet für den Einsatz in Form von Folien für den pharmazeutischen, lebensmitteltechnischen und technischen Bereich.

In schlagzähmodifizierter Form sind die erfindungsgemäß hergestellten Materialien als Strukturwerkstoff in verschiedenen technischen Bereichen einsetzbar (DE-A-42 13 219).

Die erfindungsgemäß erhaltenen Polymere sind auch für die Herstellung von Polymerlegierungen einsetzbar. Die Legierungen können in der Schmelze oder in Lösung hergestellt werden. Die Legierungen weisen jeweils eine für bestimmte Anwendungen günstige Eigenschaftskombination der Komponenten auf. Für Legierungen mit den erfindungsgemäßen Polymeren sind bevorzugt folgende Polymere einsetzbar:

Polyethylen, Polypropylen, 1-(Ethylen-Propylen)-Copolymere, Polybutylen, Poly-(4-methyl-1-penten), Polyisopren, Polyisobutylen, Naturkautschuk, Poly-1-(Methylmethacrylat), weitere Polymethacrylate, Polyacrylate, (Acrylat-Methacrylat)-Copolymere, Polystyrol, (Styrol-Acrylnitril)-Copolymere, Bisphenol-A-Polycarbonat, weitere Polycarbonate, aromatische Polyestercarbonate, Polyethylenterephthalat, Polybutylenterephthalat, amorphe Polyarylate, Nylon-6, Nylon-66, weitere Polyamide, Polyaramide, Polyetherketone, Polyoxymethylen, Polyoxyethylen, Polyurethane, Polysulfone, Polyethersulfone, Polyvinylidenfluorid.

Oberflächen von Werkstücken und Formteilen hergestellt aus den erfindungsgemäßen Cycloolefincopolymeren können durch geeignete Verfahren wie Fluorierung, Coronabehandlung, Beflammung, Plasmabehandlung modifiziert werden. Dabei lassen sich Eigenschaften wie Haftung oder Bedruckbarkeit verändern, ohne daß die Anforderung der vorliegenden Erfindung beeinträchtigt wird.

Das erfindungsgemäße Verfahren liefert transparente Cycloolefincopolymere, die hohe Molmassen aufweisen.

Die in den folgenden Beispielen angegebenen Glastemperaturen Tg wurden mittels DSC (Differential Scanning Calorimetry) bei einer Aufheizrate von 20 °C/min bestimmt. Die angegebenen Viskositätszahlen VZ wurden gemäß DIN 53728 in Decalin bei 135 °C ermittelt. Die massenmittlere Molmasse und die Polydispersität wurden mittels GPC bestimmt.

Die Erfindung wird durch folgende Beispiele näher erläutert:

### Beispiele

### Beispiel 1

In einem 1,5 dm³-Autoklav, der vorher gründlich mit Ethen gespült wurde, wurden 600 cm³ einer 85 Gew.%igen Lösung von Norbornen in Toluol vorgelegt. Durch mehrfaches Aufdrücken von Ethen (18 bar) wurde die Lösung mit Ethen gesättigt. In den so vorbereiteten Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 4,7 mg (t-Butylamido)(1-η⁵-indenyl)-dimethylsilantitandichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 18 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Nach der Trocknung wurden 29 g farbloses Polymer erhalten, welches eine Glastemperatur von 103 °C, eine Viskositätszahl von 271 ml/g, eine gewichtsmittlere Molmasse von 270.000 g/mol und eine Molmassenverteilung von 2,0 aufwies.

### Beispiel 2

In einem 1,5 dm³-Autoklav, der vorher gründlich mit Ethen gespült wurde, wurden 600 cm³ einer 85 Gew.%igen Lösung von Norbornen in Toluol vorgelegt. Durch mehrfaches Aufdrücken von Ethen (18 bar) wurde die Lösung mit Ethen gesättigt. In den so vorbereiteten Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 3,7 mg (t-Butylamido)dimethyl(3-methyl-η⁵-cyclopentadienyl)silantitandichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 18 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen., der Rückstand in Aceton aufgeschlämmt und erneut filtriert. Das so gereinigte Polymer wurde bei 80 °C im Vakuum (0,2 bar) 15 Stunden getrocknet.

Nach der Trocknung wurden 31 g farbloses Polymer erhalten, welches eine Glastemperatur von 102 °C, eine Viskositätszahl von 291 ml/g, eine gewichtsmittlere Molmasse von 253.000 g/mol und eine Molmassenverteilung von 2,1 aufwies.

### Beispiel 3

In einem 1,5 dm³-Autoklav, der vorher gründlich mit Ethen gespült wurde, wurden 600 cm³ einer 85 Gew.%igen Lösung von Norbornen in Toluol vorgelegt. Durch mehrfaches Aufdrücken von Ethen (6 bar) wurde die Lösung mit Ethen gesättigt. In den so vorbereiteten Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 7,8 mg (t-Butylamido)dimethyl(3-methyl-η⁵-cyclopentadienyl)silantitandichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 6 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polmyer wurde bei 80 °C im Vakuum (0,2 bar) 15 Stunden getrocknet.

Nach der Trocknung wurden 10 g farbloses Polymer erhalten, welches eine Glastemperatur von 122 °C, eine Viskositätszahl von 180 ml/g, eine gewichtsmittlere Molmasse von 143.000 g/mol und eine Molmassenverteilung von 1,8 aufwies.

### Beispiele 4 und 5

In einem 1,5 dm³-Autoklav, der vorher gründlich mit Ethen gespült wurde, wurden 600 cm³ einer Lösung von Norbornen in Toluol vorgelegt. Durch mehrfaches Aufdrücken von Ethen (18 bar) wurde die Lösung mit Ethen gesättigt. In den so vorbereiteten Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von (t-Butylamido)(1-η⁵-indenyl)dimethylsilantitandichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 18 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Es wurde ein farbloses Polymer enthalten. Die ergänzenden Reaktionsbedingungen und die charakteristischen Daten des Polymers sind in Tabelle 1 zusammengefaßt.

**Tabelle 1**

| Bsp. | Monomerlösung [Gew.% Norb.] | Katalysatormenge [mg] | Ausbeute [g] | Tg [°C] | VZ [ml/g] | Mw [g/mol] | Mw/Mn |
|---|---|---|---|---|---|---|---|
| 4 | 50 | 3,8 | 53 | 80 | 343 | 199.000 | 1,8 |
| 5 | 30 | 3.9 | 71 | 57 | 272 | 171.000 | 1,9 |

### Beispiele 6 und 7

In einem 1,5 dm³-Autoklav, der vorher gründlich mit Ethen gespült wurde, wurden 600 cm³ einer Lösung von Norbornen in Toluol vorgelegt. Durch mehrfaches Aufdrücken von Ethen (18 bar) wurde die Lösung mit Ethen gesättigt. In den so vorbereiteten Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von (t-Butylamido)dimethyl(3-methyl-η⁵-cyclopentadienyl)silantitandichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 18 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Es wurde ein farbloses Polymer erhalten. Die ergänzenden Reaktionsbedingungen und die charakteristischen Daten des Polymers sind in Tabelle 2 zusammengefaßt.

**Tabelle 2**

| Bsp. | Monomerlösung [Gew.% Norb.] | Katalysatormenge [mg] | Ausbeute [g] | Tg [°C] | VZ [ml/g] | Mw [g/mol] | Mw/Mn |
|---|---|---|---|---|---|---|---|
| 6 | 50 | 5,4 | 32 | 79 | 97 | 59.000 | 1,7 |
| 7 | 30 | 4.3 | 49 | 55 | 103 | 56.000 | 1.6 |

### Beispiel 8

In einem 1 dm³-Autoklav, der vorher gründlich mit Ethen gespült wurde, wurden 400 cm³ einer 85 Gew.%igen Lösung von Norbornen in Toluol vorgelegt. Durch mehrfaches Aufdrücken von Ethen (3,4 bar) wurde die Lösung mit Ethen gesättigt. In den so vorbereiteten Reaktor wurden im Gegenstrom 1 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 0,29 mg (t-Butylamido)dimethyl(tetramethyl-η⁵-cyclopentadienyl)silantitandichlorid in 1 cm³ toluolischer Methylaluminoxanlösung wurde nach 15 minütiger Voraktivierung zugegeben.

Unter Rühren (800 UPM) wurde 65 Minuten polymerisiert, wobei der Ethendruck durch Nachdosieren bei 3,4 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure dreimal mit Aceton gewaschen. Das so erhaltene Polymer wurde bei 70 °C im Vakuum (0,2 bar) 15 Stunden getrocknet.

Nach der Trocknung wurden 1,6 g farbloses Polymer erhalten, welches eine Glastemperatur von 121 °C, eine Viskositätszahl von 255 ml/g, eine gewichtsmittlere Molmasse von 313.000 g/mol und eine Molmassenverteilung von 2,5 aufwies.

### Beispiel 9

In einem 1 dm³-Autoklav, der vorher gründlich mit Ethen gespült wurde, wurden 400 cm³ einer 85 Gew.%igen Lösung von Norbornen in Toluol vorgelegt. Durch mehrfaches Aufdrücken von Ethen (6,8 bar) wurde die Lösung mit Ethen gesättigt. In den so vorbereiteten Reaktor wurden im Gegenstrom 1 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 0,29 mg (t-Butylamido)dimethyl(tetramethyl-η⁵-cyclopentadienyl)silantitandichlorid in 1 cm³ toluolischer Methylaluminoxanlösung wurde nach 15 minütiger Voraktivierung zugegeben.

Unter Rühren (800 UPM) wurde 1 Stunde polymerisiert, wobei der Ethendruck durch Nachdosieren bei 6,8 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure dreimal mit Aceton gewaschen. Das so erhaltene Polymer wurde bei 70 °C im Vakuum (0,2 bar) 15 Stunden getrocknet.

Nach der Trocknung wurden 6,9 g farbloses Polymer erhalten, welches eine Glastemperatur von 101 °C, eine Viskositätszahl von 507 ml/g, eine gewichtsmittlere Molmasse von 609.000 g/mol und eine Molmassenverteilung von 2,3 aufwies.

### Beispiele 10 bis 16

In einem 1 dm³-Autoklav, der vorher gründlich mit Ethen gespült wurde, wurden 400 cm³ einer 85 Gew.%igen Lösung von Norbornen in Toluol vorgelegt. Durch mehrfaches Aufdrücken von Ethen wurde die Lösung mit Ethen gesättigt. In den so vorbereiteten Reaktor wurden im Gegenstrom 1 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 0,29 mg (t-Butylamido)dimethyl(tetramethyl-η⁵-cyclopentadienyl)silantitandichlorid in 1 cm³ toluolischer Methylaluminoxanlösung wurde nach 15 minütiger Voraktivierung zugegeben.

Unter Rühren (800 UPM) wurde 1 Stunde polymerisiert, wobei der Ethendruck durch Nachdosieren gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure dreimal mit Aceton gewaschen. Das so erhaltene Polymer wurde bei 70 °C im Vakuum (0,2 bar) 15 Stunden getrocknet.

Nach der Trocknung wurden ein farbloses Polymer erhalten. Die entsprechenden Reaktionsbedingungen und Ausbeuten sowie die charakteristischen Daten der erhaltenen Polymere, wie Glastemperaturen, Viskositätszahlen, gewichtsmittlere Molmassen und Molmassenverteilungen gehen aus der nachfolgenden Tabelle 3 hervor.

**Tabelle 3**

| Bsp. | Ethendruck [bar] | Polymerisationszeit [min] | Ausbeute [g] | Tg [°C] | VZ [ml/g] | Mw [g/mol] | Mw/Mn |
|---|---|---|---|---|---|---|---|
| 10 | 20,5 | 8 | 8,0 | 54 | - | 810.000 | 2,1 |
| 11 | 11,0 | 40 | 6,5 | 68 | 434 | 671.000 | 2,1 |
| 12 | 15,1 | 10 | 5,3 | 55 | 542 | 733.000 | 1,8 |
| 13 | 26,8 | 7 | 13,4 | 41 | - | 850.000 | 2,0 |
| 14 | 30,8 | 7 | 13,5 | 34 | - | 819.000 | 1,7 |
| 15 | 39,2 | 4 | 20,4 | 21 | - | 1.547.000 | 2,0 |
| 16 | 50,0 | 15 | 1,7 | 11 | - | 1.384.000 | 1,9 |

### Beispiel 17

In einem 1 dm³-Autoklav, der vorher gründlich mit Ethen gespült wurde, wurden 400 cm³ einer 42 Gew.%igen Lösung von Norbornen in Toluol vorgelegt. Durch mehrfaches Aufdrücken von Ethen (44,8 bar) wurde die Lösung mit Ethen gesättigt. In den so vorbereiteten Reaktor wurden im Gegenstrom 1 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 0,15 mg (t-Butylamido)dimethyl(tetramethyl-η⁵-cyclopentadienyl)silantitandichlorid in 1 cm³ toluolischer Methylaluminoxanlösung wurde nach 15 minütiger Voraktivierung zugegeben.

Unter Rühren (800 UPM) wurde sechs Minuten polymerisiert, wobei der Ethendruck durch Nachdosieren bei 44,8 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure dreimal mit Aceton gewaschen. Das so erhaltene Polymer wurde bei 70 °C im Vakuum (0,2 bar) 15 Stunden getrocknet.

Nach der Trocknung wurden 10,3 g farbloses Polymer erhalten, welches eine Glastemperatur von -2 °C, eine gewichtsmittlere Molmasse von 1.420.000 g/mol und eine Molmassenverteilung von 1,9 aufwies.

### Vergleichsbeispiel 1

In einem 1,5 dm³-Autoklav, der vorher gründlich mit Ethen gespült wurde, wurden 1000 cm³ einer 85 Gew.%igen Lösung von Norbornen in Toluol vorgelegt. Durch mehrfaches Aufdrücken von Ethen (6 bar) wurde die Lösung mit Ethen gesättigt. In den so vorbereiteten Reaktor wurden im Gegenstrom 20 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 48 mg Dimethylsilylbis(cyclopentadienyl)zirkondichlorid in 20 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 6 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Nach der Trocknung wurden 104 g farbloses Polymer erhalten, welches ein Glastemperatur von 214 °C und eine Viskositätszahl von 27 ml/g, eine gewichtsmittlere Molmasse von 25.000 g/mol und eine Molmassenverteilung von 2,0 aufwies.

### Vergleichsbeispiel 2

In einem 1,5 dm³-Autoklav, der vorher gründlich mit Ethen gespült wurde, wurden 600 cm³ einer 85 Gew.%igen Lösung von Norbornen in Toluol vorgelegt. Durch mehrfaches Aufdrücken von Ethen (16 bar) wurde die Lösung mit Ethen gesättigt. In den so vorbereiteten Reaktor wurden im Gegenstrom 20 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 4,6 mg Dimethylsilylbis(cyclopentadienyl)zirkondichlorid in 20 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 16 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Nach der Trocknung wurden 49 g farbloses Polymer erhalten, welches ein Glastemperatur von 177 °C, eine Viskositätszahl von 56 ml/g, eine gewichtsmittlere Molmasse von 47.000 g/mol und eine Molmassenverteilung von 2,0 aufwies.

### Vergleichsbeispiele 3 und 4

In einem 1,5 dm³-Autoklav, der vorher gründlich mit Ethen gespült wurde, wurden 600 cm³ einer 85 Gew.%igen Lösung von Norbornen in Toluol vorgelegt. Durch mehrfaches Aufdrücken von Ethen wurde die Lösung mit Ethen gesättigt. In den so vorbereiteten Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 1 mg Isopropylen(cyclopentadienyl)(1-indenyl)zirkondichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wurde je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Es wurde ein farbloses Polymer enthalten. Die ergänzenden Reaktionsbedingungen und die charakteristischen Daten des Polymers sind in Tabelle 4 zusammengefaßt.

**Tabelle 4**

| Vergleichsbeispiel | Monomerlösung [Gew.% Norb.] | Ethylendruck [bar] | Ausbeute [g] | Tg [°C] | VZ [ml/g] | Mw [g/mol] | Mw/Mn |
|---|---|---|---|---|---|---|---|
| 3 | 50 | 10 | 44 | 163 | 70 | 62.000 | 2,0 |
| 4 | 30 | 12 | 116 | 120 | 63 | 56.000 | 2,1 |

### Vergleichsbeispiele 5 bis 7

In einem 70 dm³-Autoklav, der vorher gründlich mit Ethen gespült wurde, wurde eine Lösung von Norbornen in Toluol vorgelegt. Durch mehrfaches Aufdrücken von Ethen wurde die Lösung mit Ethen gesättigt. In den so vorbereiteten Reaktor wurden im Gegenstrom 400 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von lsopropylen(cyclopentadienyl)(1-indenyl)zirkondichlorid in 300 cm³ toluolischer Methylaluminoxanlösung wurde nach 30 minütiger Voraktivierung zugegeben.

Unter Rühren (750 UPM) wurde eine Stunde polymerisiert, wobei der Ethendruck durch Nachdosieren gehalten wurde.

Nach Ende der Reaktionszeit wurde das Polymerisationsgemisch in ein Gefäß abgelassen und sofort in 300 dm³ Aceton eingetragen, 30 Minuten gerührt und anschließend das ausgefallene Produkt filtriert. Der Filterkuchen wurde je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Es wurde ein farbloses Polymer enthalten. Die ergänzenden Reaktionsbedingungen und die charakteristischen Daten des Polymers sind in Tabelle 5 und 6 zusammengefaßt.

**Tabelle 5**

| Vergleichsbeispiel | Monomerlösung [Gew.% Norb.] | Volumen Monomerlösung [dm³] | Katalysatormenge [mg] | Ethylendruck [bar] |
|---|---|---|---|---|
| 5 | 60 | 30 | 100 | 20 |
| 6 | 40 | 50 | 160 | 22 |
| 7 | 40 | 32 | 100 | 20 |

**Tabelle 6**

| Vergleichsbeispiel | Ausbeute [g] | T_{g} [° C] | VZ [ml/g] | M_{w} [g/mol] | M_{w}/Mₙ |
|---|---|---|---|---|---|
| 5 | 8 | 105 | 47 | 29.000 | 1,9 |
| 6 | 7,4 | 89 | 41 | 21.000 | 1,9 |
| 7 | 7 | 82 | 38 | 21.000 | 1.8 |

### Vergleichsbeispiel 8

In einem 1,5 dm³-Autoklav, der vorher gründlich mit Ethen gespült wurde, wurden 600 cm³ einer 80 Gew.%igen Lösung von Norbornen in Toluol vorgelegt. Durch mehrfaches Aufdrücken von Ethen (3 bar) wurde die Lösung mit Ethen gesättigt. In den so vorbereiteten Reaktor wurden im Gegenstrom 20 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 50 mg lsopropylen(3-methylcyclopentadienyl)(1-indenyl)zirkondichlorid in 20 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 3 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wure je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Nach der Trocknung wurden 43 g farbloses Polymer erhalten, welches eine Glastemperatur von 174 °C und eine Viskositätszahl von 73 ml/g aufwies.

### Vergleichsbeispiele 9 bis 12

In einem 1,5 dm³-Autoklav, der vorher gründlich mit Ethen gespült wurde, wurde eine Lösung von 600 cm³ Norbornen in Toluol vorgelegt. Durch mehrfaches Aufdrücken von Ethen wurde die Lösung mit Ethen (6 bar) gesättigt. In den so vorbereiteten Reaktor wurden im Gegenstrom 20 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von Dimethylsilylbis(1-indenyl)zirkondichlorid in 20 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren bei 6 bar gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wurde je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Es wurde ein farbloses Polymer enthalten. Die ergänzenden Reaktionsbedingungen und die charakteristischen Daten des Polymers sind in Tabelle 7 zusammengefaßt.

**Tabelle 7**

| Vergleichsbeispiel | Monomerlösung [Gew.% Norb.] | Katalysatormenge [mg] | Ausbeute [g] | Tg [°C] | Tm [°C] | VZ [ml/g] |
|---|---|---|---|---|---|---|
| 9 | 85 | 5 | 21 | 122 | 122 | 45 |
| 10 | 85 | 5 | 20 | 178 | 118 | 65 |
| 11 | 80 | 0,5 | 2,5 | 138 | 120 | 45 |
| 12 | 30 | 60 | 180 | 103 | 115 | 86 |

### Vergleichsbeispiel 13 bis 16

In einem 1,5 dm³-Autoklav, der vorher gründlich mit Ethen gespült wurde, wurde eine Lösung von 600 cm³ Norbornen in Toluol vorgelegt. Durch mehrfaches Aufdrücken von Ethen wurde die Lösung mit Ethen gesättigt. In den so vorbereiteten

Reaktor wurden im Gegenstrom 10 cm³ toluolische Methylaluminoxanlösung (10 Gew.%ige Methylaluminoxanlösung der Molmasse 1300 g/mol nach kryoskopischer Bestimmung) dosiert und 30 Minuten bei 70 °C gerührt. Eine Lösung von 1,8 mg Dimethylsilyl(cyclopentadienyl)(1-indenyl)zirkondichlorid in 10 cm³ toluolischer Methylaluminoxanlösung wurde nach 15 minütiger Voraktivierung zugegeben.

Unter Rühren (750 UPM) wurde eine Stunde polymerisiert, wobei der Ethendruck durch Nachdosieren gehalten wurde.

Nach Ende der Reaktionszeit wurde das Polymerisationsgemisch in ein Gefäß abgelassen und sofort in 5 dm³ Aceton eingetragen, 10 Minuten gerührt und anschließend das ausgefallene Produkt filtriert. Der Filterkuchen wurde je dreimal abbwechselnd mit 10%iger Salzsäure und Aceton gewaschen, der Rückstand in Aceton aufgeschlämmt und erneut filtriert. Das so gereinigte Polymer wurde bei 80 °C im Vakuum (0,2 bar) 15 Stunden getrocknet.

Es wurde ein farbloses Polymer enthalten. Die ergänzenden Reaktionsbedingungen und die charakteristischen Daten des Polymers sind in Tabelle 8 zusammengefaßt.

**Tabelle 8**

| Vergleichsbeispiel | Monomerlösung [Gew% Norb.] | Ethylendruck [bar] | Ausbeute [g] | Tg [°C] | VZ [ml/g] |
|---|---|---|---|---|---|
| 13 | 85 | 6 | 9,4 | 214 | 68 |
| 14 | 85 | 18 | 14,6 | 161 | 130 |
| 15 | 50 | 18 | 33,9 | 113 | 132 |
| 16 | 30 | 18 | 46.8 | 68 | 130 |

## Patentansprüche

1. Verfahren zur Herstellung eines Cycloolefincopolymers durch Polymerisation von 0,1 bis 99.9 Gew.-%, bezogen auf die Gesamtmenge der Monomeren, mindestens eines polycyclischen Olefins, 0 bis 99,9 Gew.-%, bezogen auf die Gesamtmenge der Monomeren, mindestens eines monocyclischen Olefins und 0,1 bis 99,9 Gew.-%, bezogen auf die Gesamtmenge der Monomeren, mindestens eines acyclischen 1-Olefins, in Gegenwart eines Katalysatorssystems, welches aus mindestens einem Cokatalysator und aus mindestens einem Metall-Koordinations-Komplex gespannter Geometrie der Formel (I) besteht 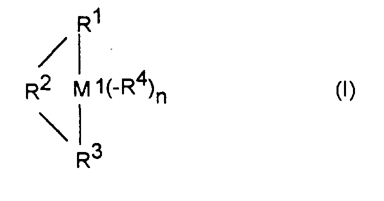 wobei
M¹ ein Metall der Gruppe 3 bis 10 oder der Lanthaniden-Reihe des Periodensystems der Elemente ist,
R¹ ein delokalisiertes acyclisches n-System wie C₄-C₂₀-Alkenyl, C₄-C₂₀-Alkinyl, C₃-C₂₀-Allyl, C₄-C₂₀-Alkadienyl, C₄-C₂₀-Polyenyl oder vergleichbare Strukturen, die bis zu 5 Heteroatomen enthalten können, oder ein unsubstituiertes oder substituiertes delokalisiertes C₅-C₄₀-cylisches π-System oder vergleichbare Strukturen, die bis zu 5 Heteroatomen enthalten können, bedeutet,
R² eine ein- oder mehrgliedrige Brücke ist, welche die Reste R¹ und R³ verknüpft und mindestens ein Atom der Gruppe 14 des Periodensystems der Elemente oder mindestens ein Bor-Atom enthält und ein oder mehrere Schwefel- oder Sauerstoffatome enthalten kann und mit R¹ ein kondensiertes Ringsystem bilden kann,
R³ einen anionischen oder nichtionischen Liganden bedeutet, der an M¹ koordiniert ist und ein oder mehrere Stickstoff-, Phosphor-, Sauerstoff-und/ oder Schwefelatome enthält und mit R² ein kondensiertes Ringsystem bilden kann, und
R⁴ ein anionischer oder nichtionischer Ligand ist, wobei n = 0, 1, 2, 3 oder 4 in Abhängigkeit von der Valenz von M bedeutet, **dadurch gekennzeichnet, dass** der Metall-Koordinations-Komplex vor dem Einsatz in der polymerisations reaktion mit dem Cokatalysation voraktiviert wird, mit der Massgabe, dass Metall-Koordinations Komplexe, worin R¹ ein asymmetrisch substituierter Monocyclopentadienylligand ist, und dass die Verbindung Dimethylsilyl-(tetrametyl cyclopentadienyl)-(tert-butyl amido)-dimethyl hafnium ausgeschlossen sind.

2. Verfahren gemäß Anspruch 1, wobei der Metall-Koordinations-Komplex eine Verbindung der Formel (la) bedeutet: 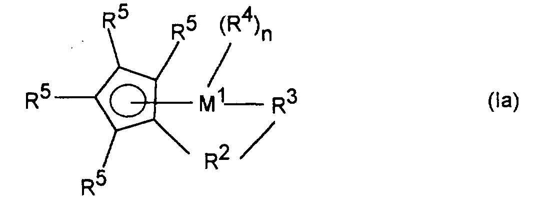 wobei
M¹ ein Metall der Gruppe 4 oder der Lanthaniden-Reihe des Periodensystems der Elemente ist,
R² eine ein- oder mehrgliedrige Brücke ist, welche das η⁵-koordinierte cyclische π-System und R³ verknüpft, und bedeutet vorzugsweise 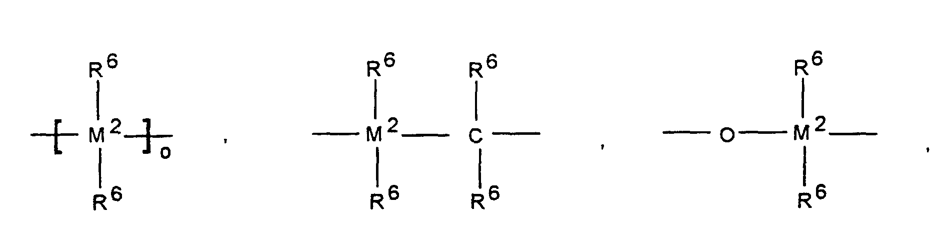 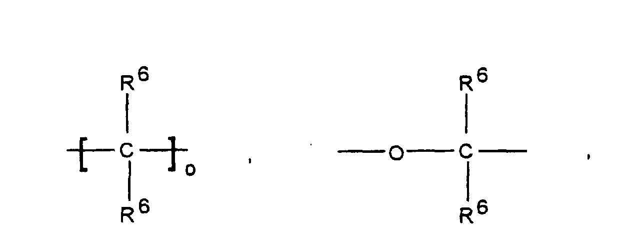 =BR⁶, =AIR⁶, -Ge-, -Sn-, -O-, -S-, =SO, =SO₂, =NR⁶, =CO, =PR⁶, oder =P(O)R⁶, wobei R⁶ gleich oder verschieden sind und ein Wasserstoffatom, ein Halogenatom, eine C₁-C₄₀-kohlenstoffhaltige Gruppe wie eine C₁-C₁₀-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe, -SiR⁷₃, -NR⁷₂, -Si(OR⁷)₃,-Si(SR⁷)₃ oder -PR⁷₂ bedeuten,
worin R⁷ gleich oder verschieden ein Halogenatom, eine C₁-C₁₀-Alkylgruppe oder eine C₆-C₁₀-Arylgruppe sind oder ein Ringsystem bilden, wobei o ≥ 1 ist, und
M² Silicium, Germanium oder Zinn ist,
R³ O, S, NR⁸, PR⁸ oder einen neutralen 2-Elektronen-Donor-Liganden, ausgewählt aus der Gruppe OR⁸, SR⁸, NR⁸₂, PR⁸₂, wobei R⁸ ein Wasserstoffatom, ein Halogenatom, eine C₁-C₄₀-kohlenstoffhaltige Gruppe wie eine C₁-C₈-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe, -SiR⁹₃, -NR⁹₂, -Si(OR⁹)₃,-Si(SR⁹)₃ oder -PR⁹₂ bedeuten, worin R⁹ gleich oder verschieden ein Halogenatom, eine C₁-C₈-Alkylgruppe oder eine C₆-C₁₀-Arylgruppe sind,
R⁴ gleich oder verschieden sind und ein Wasserstoffatom, eine C₁-C₄₀-kohlenstoffhaltige Gruppe wie eine C₁-C₁₀-Alkyl-, eine C₁-C₁₀-Alkoxy-, eine C₆-C₁₀-Aryl-, eine C₆-C₂₅-Aryloxy-, eine C₂-C₁₀-Alkenyl-, eine C₇-C₄₀-Arylalkyl- oder eine C₇-C₄₀-Arylalkenylgruppe, eine OH-Gruppe, ein Halogenatom oder NR¹⁰₂, worin R¹⁰ ein Halogenatom, eine C₁-C₁₀-Alkylgruppe oder eine C₆-C₁₀-Arylgruppe sind, bedeuten, oder R⁴ zusammen mit den sie verbindenden Atomen ein Ringsystem bilden, wobei n = 1 oder 2 ist,
R⁵ gleich oder verschieden sind und ein Wasserstoffatom, ein Halogenatom, eine C₁-C₄₀-kohlenstoffhaltige Gruppe wie eine C₁-C₁₀-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, oder eine C₈-C₄₀-Arylalkenylgruppe, -SiR¹¹₃, -NR¹¹₂, -Si(OR¹¹)₃,-Si(SR¹¹)₃ oder -PR¹¹₂ bedeuten, worin R¹¹ gleich oder verschieden ein Halogenatom, eine C₁-C₁₀-Alkylgruppe oder eine C₆-C₁₀-Arylgruppe sind oder ein Ringsystem bilden, oder zwei oder mehr benachbarte Substituenten R⁵ zusammen mit den sie verbindenden Atomen ein Ringsystem bilden, welches bevorzugt 4 bis 40, besonders bevorzugt 6 bis 20 Koblenstoffatome enthält

3. Verfahren gemäß einem oder mehreren der Ansprüche 1 bis 2, wobei der Metall-Koordinations-Komplex eine Verbindung der Formel (lb) ist: 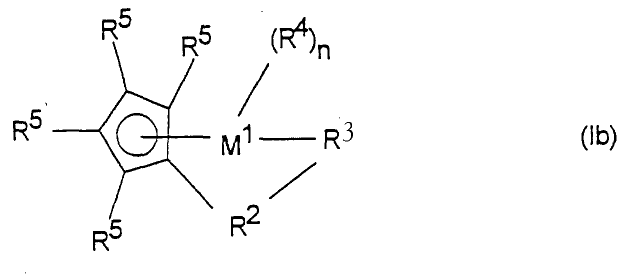 wobei
M¹ Titan bedeutet,
R² eine ein-, zwei- oder dreigliedrige Brücke ist, welche das η⁵-koordinierte cyclische π-System und R³ verknüpft, und bedeutet vorzugsweise 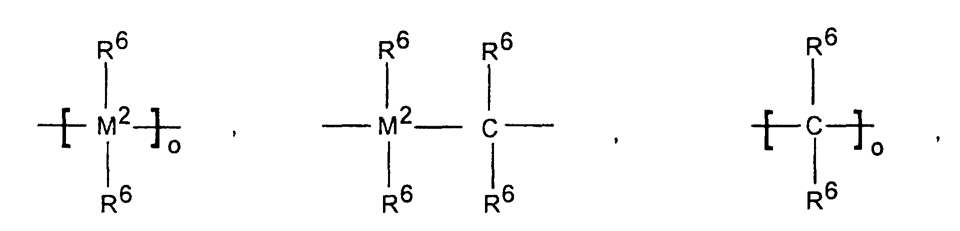 wobei R⁶ gleich oder verschieden sind und ein Wasserstoffatom, eine C₁-C₄₀-kohlenstoffhaltige Gruppe wie eine C₁-C₁₀-Alkylgruppe, die halogeniert sein kann, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe bedeutet, wobei o = 1, 2 oder 3 ist, und
M² Silicium ist,
R³ NR⁸ ist, wobei R⁸ ein Wasserstoffatom, eine C₁-C₄₀-kohlenstoffhaltige Gruppe wie eine C₁-C₈-Alkylgruppe, die halogeniert sein kann, C₁-C₁₀-Alkoxygruppe, eine C₆-C₂₀-Arylgruppe, die halogeniert sein kann, eine C₆-C₂₀-Aryloxy-, eine C₂-C₁₂-Alkenyl-, eine C₇-C₄₀-Arylalkyl-, eine C₇-C₄₀-Alkylaryl-, eine C₈-C₄₀-Arylalkenylgruppe ist,
R⁴ gleich oder verschieden sind und ein Wasserstoffatom, eine C₁-C₄₀-kohlenstoffhaltige Gruppe wie eine C₁-C₁₀-Alkyl-, eine C₁-C₁₀-Alkoxy-, eine C₆-C₁₀-Aryl-, eine C₆-C₂₅-Aryloxy-, eine C₂-C₁₀-Alkenyl-, eine C₇-C₄₀-Arylalkyl- oder eine C₇-C₄₀-Arylalkenylgruppe, eine OH-Gruppe, ein Halogenatom oder NR¹⁰₂, worin R¹⁰ ein Halogenatom, eine C₁-C₁₀-Alkylgruppe oder eine C₆-C₁₀-Arylgruppe sind, bedeuten, oder zusammen mit den sie verbindenden Atomen ein Ringsystem bilden,
R⁵ gleich oder verschieden sind und ein Wasserstoffatom, eine C₁-C₁₀-Alkyl- oder Trimethylsilyl-Gruppe bedeuten oder zwei der Substituenten R⁵ mit dem sie verbindenden Cyclopentadienyl-System einen sechsgliedrigen aromatischen kondensierten Ring bilden.

4. Verfahren gemäß einem oder mehreren der Ansprüche 1 bis 3, wobei der Metall-Koordinations-Komplex der Formel (I) eine Verbindung ausgewählt aus den Verbindungen
(t-Butylamido)(tetramethyl-η⁵-cyclopentadienyl)dimethylsilantitandichlorid
(Phenylamido)(tetramethyl-η⁵-cyclopentadienyl)dimethylsilantitandichlorid
(Cyclohexylamido)(tetramethyl-η⁵-cyclopentadienyl)dimethylsilantitandichlorid
(t-Butylamido)(1-η⁵-indenyl)dimethylsilantitandichlorid
(Phenylamido)(1-η⁵-indenyl)dimethylsilantitandichlorid
(Phenylamido)(1-η⁵-indenyl)dimethylsilantitandichlorid
(Cyclohexylamido)(1-η⁵-indenyl)dimethylsilantitandichlorid
(t-Butylamido)(9-η⁵-fluorenyl)dimethylsilantitandichlorid
(Phenylamido)(9-η⁵-fluorenyl)dimethylsilantitandichlorid
(Cyclohexylamido)(9-η⁵-fluorenyl)dimethylsilantitandichlorid ist.

5. Verfahren gemäß einem oder mehreren der Ansprüche 1 bis 4, wobei als Cokatalysator ein Aluminoxan eingesetzt wird.

6. Verfahren gemäß einem oder mehreren der Ansprüche 1 bis 5, durch Polymerisation von 0,1 bis 99,9 Gew.-%, bezogen auf die Gesamtmenge der Monomeren, mindestens eines polycyclischen Olefins der Formel III, IV, IV', V, VI, VII oder VIII 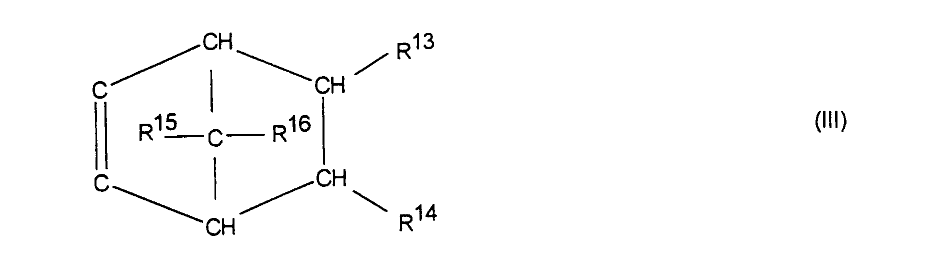 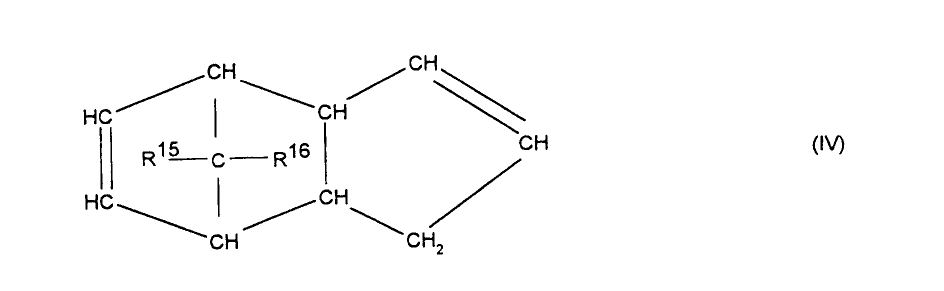 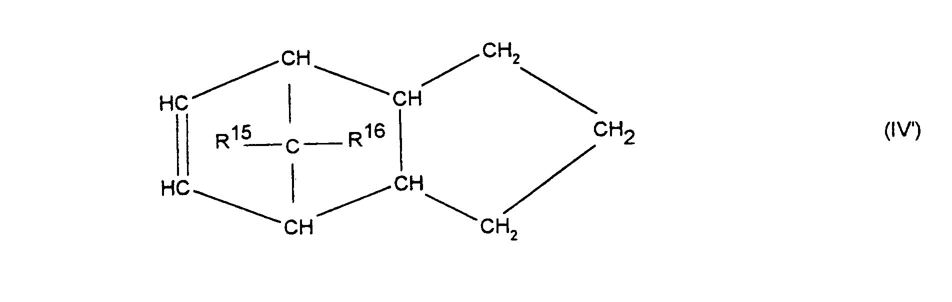 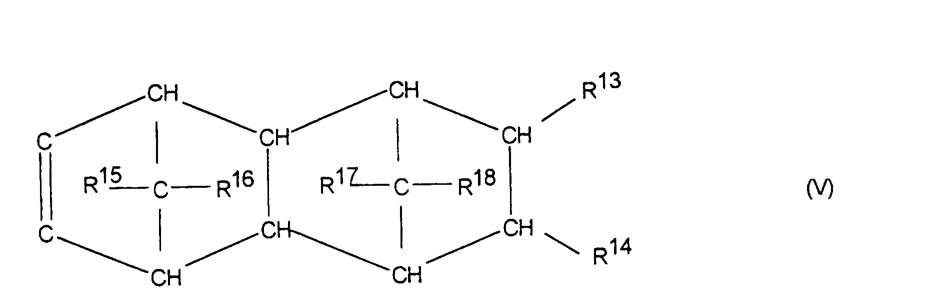 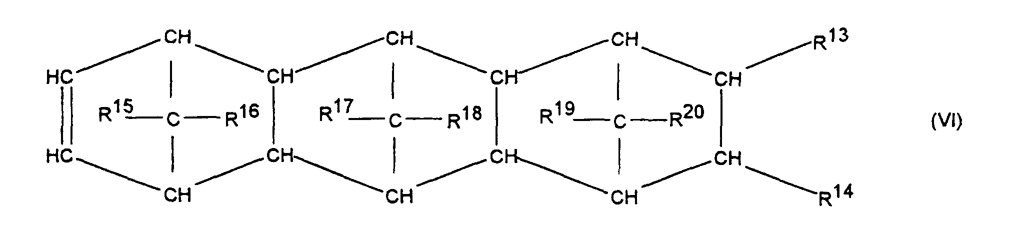 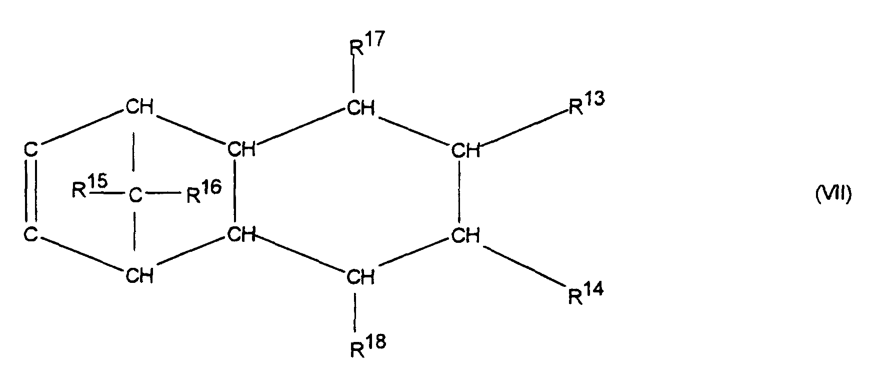 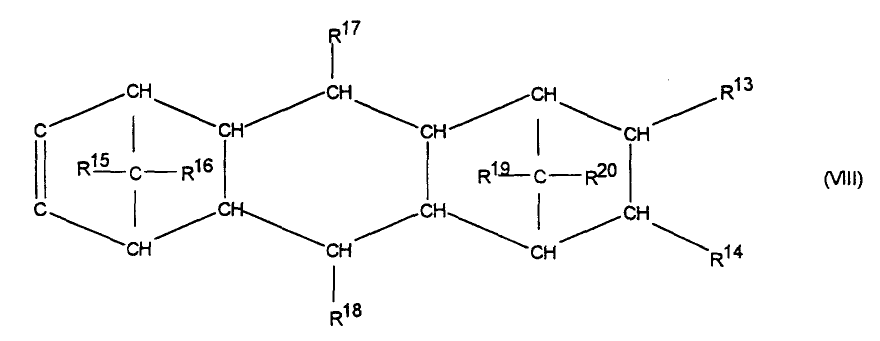 worin R¹³, R¹⁴, R¹⁵, R¹⁶, R¹⁷, R¹⁸, R¹⁹ und R²⁰ gleich oder verschieden sind und ein Wasserstoffatom oder einen Kohlenwasserstoffrest bedeuten, wobei gleiche Reste in den verschiedenen Formeln eine unterschiedliche Bedeutung haben können, 0 bis 99,9 Gew.-%, bezogen auf die Gesamtmenge der Monomeren, mindestens eines monocyclischen Olefins der Formel IX 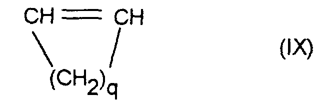 worin q eine Zahl von 2 bis 10 ist, und 0,1 bis 99,9 Gew.-%, bezogen auf die Gesamtmenge der Monomeren, mindestens eines acyclischen 1-Olefins der Formel X 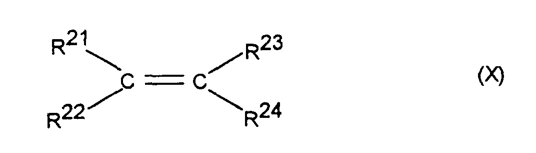 worin R²¹, R²², R²³ und R²⁴ gleich oder verschieden sind und ein Wasserstoffatom oder einen Kohlenwasserstoffrest, bevorzugt einen C₆-C₁₀-Arylrest oder einen C₁-C₈-Alkylrest bedeuten.

7. Verfahren gemäß einem oder mehreren der Ansprüche 1 bis 6, wobei das polycyclische Olefin eine Verbindung der Formeln III oder V ist, worin R¹³, R¹⁴, R¹⁵, R¹⁶, R¹⁷, R¹⁸, R¹⁹ und R²⁰ gleich oder verschieden sind und ein Wasserstoffatom oder einen Kohlenwasserstoffrest, insbesondere einen C₆-C₁₀-Arylrest oder einen C₁-C₈-Alkylrest bedeuten, wobei gleiche Reste in den verschiedenen Formeln eine unterschiedliche Bedeutung haben können.

8. Verfahren gemäß einem oder mehreren der Ansprüche 1 bis 7, wobei das polycyclische Olefin Norbornen oder Tetracyclododecen ist.

9. Verfahren gemäß einem oder mehreren der Ansprüche 1 bis 8, wobei das acyclische 1-Olefin Ethylen ist.

10. Verfahren gemäß einem oder mehreren der Ansprüche 1 bis 9, wobei das Cycloolefincopolymere mindestens 5 Mol-% Einheiten abgeleitet von dem Cycloolefin enthält.

11. Verfahren gemäß einem oder mehreren der Ansprüche 1 bis 10, wobei das Cycloolefincopolymer aus Einheiten besteht, die abgeleitet sind von Monomeren, die neben der für die Polymerisation erforderlichen olefinischen Doppelbindung keine weiteren ungesättigten Funktionalitäten aufweisen.

12. Verfahren gemäß einem oder mehreren der Ansprüche 1 bis 11, wobei eine Temperatur von -78 bis 150 °C und ein Druck von 0,01 bis 64 bar eingestellt wird.

13. Verfahren gemäß einem oder mehreren der Ansprüche1 bis 12, wobei eine Temperatur von 0 bis 100 °C und ein Druck von 0,01 bis 64 bar eingestellt wird.

14. Verfahren gemäß einem oder mehreren der Ansprüche 1 bis 13, wobei die Polymerisation im flüssigen Cycloolefin selbst oder in einer Cycloolefin-Lösung durchgeführt wird.

15. Verfahren gemäß einem oder mehreren der Anspruche 1 bis 14, wobei die Veraktivierungszeit 5 bis 60 Minuten beträgt.

16. Cycloolefincopolymer ethältlich durch Polymerisation von mindestens einem polycyclischen Olefin gegebenenfalls, mindestens einem monocyclischen Olefins und mindestens einem acyclischen 1-Olefin, wobei die Einbanrate cyclischen Komponenten 15 bis 60 Mol% beträgt, in Gegenwart eines Katalysatorssystems, welches aus mindestens einem Cokatalysator und aus mindestens einem Metall-Koordinations-Komplex gespannter Geometrie der Formel (I) besteht 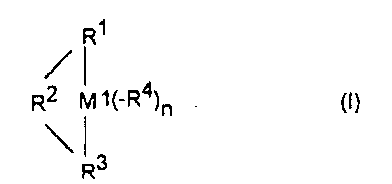 wobei
M¹ ein Metall der Gruppe 3 bis 10 oder der Lanthaniden-Reihe des Periodensystems der Elemente ist,
R¹ ein delokalisiertes acyclisches π-System wie C₄-C₂₀-Alkenyl, C₄-C₂₀-Alkinyl, C₃-C₂₀-Allyl, C₄-C₂₀-Alkadienyl, C₄-C₂₀-Polyenyl oder vergleichbare Strukturen, die bis zu 5 Heteroatomen enthalten können, oder ein unsubstituiertes oder substituiertes delokalisiertes C₅-C₄₀-cylisches n-System oder vergleichbare Strukturen, die bis zu 5 Heteroatomen enthalten können, bedeutet,
R² eine ein- oder mehrgliedrige Brücke ist, welche die Reste R¹ und R³ verknüpft und mindestens ein Atom der Gruppe 14 des Periodensystems der Elemente oder mindestens ein Bor-Atom enthält und ein oder mehrere Schwefel- oder Sauerstoffatome enthalten kann und mit R¹ ein kondensiertes Ringsystem bilden kann,
R³ einen anionischen oder nichtionischen Liganden bedeutet, der an M¹ koordiniert ist und ein oder mehrere Stickstoff-, Phosphor-, Sauerstoff-und/ oder Schwefelatome enthält und mit R² ein kondensiertes Ringsystem bilden kann, und
R⁴ ein anionischer oder nichtionischer Ligand ist, wobei n = 0, 1, 2, 3 oder 4 in Abhängigkeit von der Valenz von M bedeutet,

17. Verwendung eines Voraktivierten Katalysatorsystems enthaltend einen Metall-Koordinations-Komplex mit der Formel (I) nach Auspruch 1 zur Herstellung eines Cycloolefincopolymers.

18. Verwendung eines Cycloolefincopolymers nach Anspruch 16 zur Herstellung von Formkörpern, wie Extrusionsteilen wie Folien, Platten, Schläuchen, Rohren, Stangen und Fasern oder Spritzgußartikeln beliebiger Form und Größe.

## Claims

1. A process for preparing a cycloolefin copolymer by polymerization of from 0.1 to 99.9% by weight, based on the total amount of monomers, of at least one polycyclic olefin, from 0 to 99.9% by weight, based on the total amount of monomers, of at least one monocyclic olefin and from 0.1 to 99.9% by weight, based on the total amount of monomers, of at least one acyclic 1-olefin in the presence of a catalyst system comprising at least one cocatalyst and at least one metal complex having a strained geometry and the formula (I) 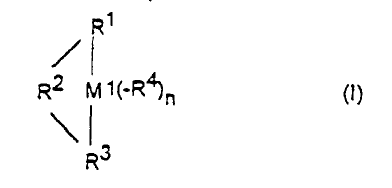 where
M¹ is a metal of groups 3 to 10 or of the lanthanide series of the Periodic Table of the Elements,
R¹ is a delocalized acyclic π system such as C₄-C₂₀-alkenyl, C₄-C₂₀-alkynyl, C₃-C₂₀-allyl, C₄-C₂₀-alkadienyl, C₄-C₂₀-polyenyl or a comparable structure which may comprise up to 5 hetero atoms, or an unsubstituted or substituted delocalized C₅-C₄₀-cyclic π system or a comparable structure which may comprise up to 5 hetero atoms,
R² is a single- or multi-membered bridge which links the radicals R¹ and R³ and comprises at least one atom of group 14 of the Periodic Table of the Elements or at least one boron atom and may comprise one or more sulfur or oxygen atoms and can form a fused ring system together with R¹,
R³ is an anionic or nonionic ligand which is coordinated to M¹ and comprises one or more nitrogen, phosphorus, oxygen and/or sulfur atoms and can form a fused ring system together with R², and
R⁴ is an anionic or nonionic ligand, where n = 0, 1, 2, 3 or 4 depending on the valence of M, wherein the metal complex is preactivated by means of the cocatalyst prior to use in the polymerization reaction, with the exception of metal complexes in which R¹ is an unsymmetrically substituted monocyclopentadienyl ligand and of the compound dimethylsilyl (tetramethylcyclopentadienyl)(tert-butyl-amido)dimethylhafnium.

2. The process as claimed in claim 1, wherein the metal complex is a compound of the formula (la): 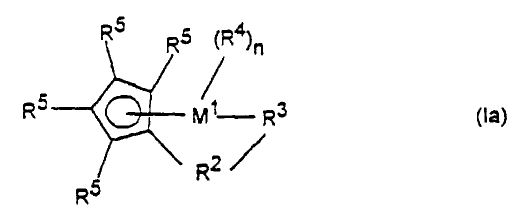 where
M¹ is a metal of group 4 or of the lanthanide series of the Periodic Table of the Elements,
R² is a single- or multi-membered bridge which links the η⁵-coordinated cyclic π system and R³ and is preferably 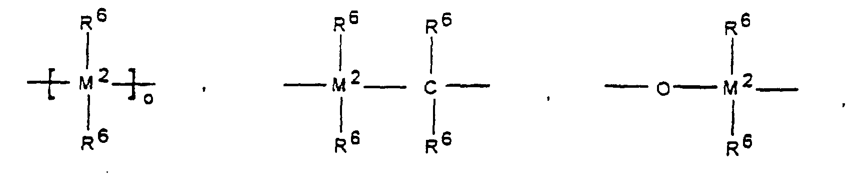 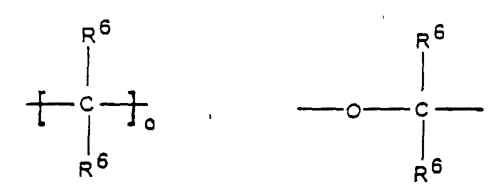 = BR⁶, = AIR⁶, -Ge-, -Sn-, -O-, -S-, =SO, =SO₂, = NR⁶, = CO, = PR⁶ or = P(O)R⁶, where R⁶ are identical or different and are each a hydrogen atom, a halogen atom, a C₁-C₄₀-group such as a C₁-C₁₀-alkyl group which may be halogenated, a C₆-C₂₀-aryl group which may be halogenated, a C₆-C₂₀-aryloxy group, a C₂-C₁₂-alkenyl group, a C₇-C₄₀-arylalkyl group, a C₇-C₄₀-alkylaryl group, a C₈-C₄₀-arylalkenyl group, -SiR⁷₃, -NR⁷₂, -Si(OR⁷)₃, -Si(SR⁷)₃ or -PR⁷₂, where R⁷ are identical or different and are each a halogen atom, a C₁-C₁₀-alkyl group or a C₆-C₁₀-aryl group or form a ring system, where o ≥ 1, and
M² is silicon, germanium or tin,
R³ is O, S, NR⁸, PR⁸ or an uncharged 2-electron donor ligand selected from the group consisting of OR , SR⁸, NR⁸₂, PR⁸₂, where R⁸ is a hydrogen atom, a halogen atom, a C₁-C₄₀-group such as a C₁-C₈-alkyl group which may be halogenated, a C₆-C₂₀-aryl group which may be halogenated, a C₆-C₂₀-aryloxy group, a C₂-C₁₂-alkenyl group, a C₇-C₄₀-arylalkyl group, a C₇-C₄₀-alkylaryl group, a C₈-C₄₀-arylalkenyl group, -SiR⁹₃, -NR⁹₂, -Si(OR⁹)₃, -Si(SR⁹)₃ or -PR⁹₂, where R⁹ are identical or different and are each a halogen atom, a C₁-C₈-alkyl group or a C₆-C₁₀-aryl group.
R⁴ are identical or different and are each a hydrogen atom, a C₁-C₄₀-group such as a C₁-C₁₀-alkyl group, a C₁-C₁₀-alkoxy group, a C₆-C₁₀-aryl group, a C₆-C₂₅-aryloxy group, a C₂-C₁₀-alkenyl group, a C₇-C₄₀-arylalkyl group or a C₇-C₄₀-arylalkenyl group, an OH group, a halogen atom or NR¹⁰₂, where R¹⁰ is a halogen atom, a C₁-C₁₀-alkyl group or a C₆-C₁₀-aryl group, or R⁴ together with the atoms connecting them form a ring system, where n = 1 or 2,
R⁵ are identical or different and are each a hydrogen atom, a halogen atom, a C₁-C₄₀-group such as a C₁-C₁₀-alkyl group which may be halogenated, a C₆-C₂₀-aryl group which may be halogenated, a C₆-C₂₀-aryloxy group, a C₂-C₁₂-alkenyl group, a C₇-C₄₀-arylalkyl group, a C₇-C₄₀-alkylaryl group or a C₈-C₄₀-arylalkenyl group, -SiR¹¹₃, -NR¹¹₂, -Si(OR¹¹)₃, -Si(SR¹¹)₃ or -PR¹¹₂, where R are identical or different and are each a halogen atom, a C₁-C₁₀-alkyl group or a C₆-C₁₀-aryl group or form a ring system, or two or more adjacent substituents R⁵ together with the atoms connecting them form a ring system which preferably comprises from 4 to 40, particularly preferably from 6 to 20, carbon atoms.

3. The process as claimed in claim 1 or 2, wherein the metal complex is a compound of the formula (1b) 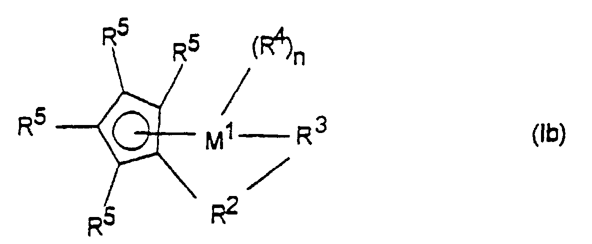 where
M¹ is titanium,
R² is a single-, two- or three-membered bridge which links the η⁵-coordinated cyclic π system and R³ and is preferably 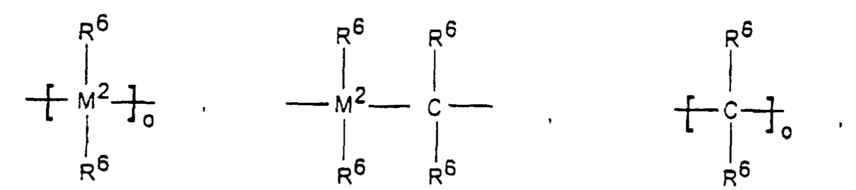 where R⁶ are identical or different and are each a hydrogen atom, a C₁-C₄₀-group such as a C₁-C₁₀-alkyl group which may be halogenated, a C₆-C₂₀-aryl group which may be halogenated, a C₆-C₂₀-aryloxy group, a C₂-C₁₂-alkenyl group, a C₇-C₄₀-arylalkyl group, a C₇-C₄₀-alkylaryl group or a C₈-C₄₀-arylalkenyl group, where o = 1, 2 or 3, and
M² is silicon,
R³ is NR⁸, where R⁸ is a hydrogen atom, a C₁-C₄₀-group such as a C₁-C₈-alkyl group which may be halogenated, a C₁-C₁₀-alkoxy group, a C₆-C₂₀-aryl group which may be halogenated, a C₆-C₂₀-aryloxy group, a C₂-C₁₂-alkenyl group, a C₇-C₄₀-arylalkyl group, a C₇-C₄₀-alkylaryl group or a C₈-C₄₀-arylalkenyl group,
R⁴ are identical or different and are each a hydrogen atom, a C₁-C₄₀-group such as a C₁-C₁₀-alkyl group, a C₁-C₁₀-alkoxy group, a C₆-C₁₀-aryl group, a C₆-C₂₅-aryloxy group, a C₂-C₁₀-alkenyl group, a C₇-C₄₀-arylalkyl group or a C₇-C₄₀-arylalkenyl group, an OH group, a halogen atom or NR¹⁰₂, where R¹⁰ is a halogen atom, a C₁-C₁₀-alkyl group or a C₆-C₁₀-aryl group, or together with the atoms connecting them form a ring system,
R⁵ are identical or different and are each a hydrogen atom, a C₁-C₁₀-alkyl group or trimethylsilyl group or two of the substituents R⁵ together with the cyclopentadienyl system connecting them form a six-membered aromatic fused ring.

4. The process as claimed in one or more of claims 1 to 3, wherein the metal complex of the formula (I) is a compound selected from among the compounds
(t-butylamido)(tetramethyl-η⁵-cyclopentadienyl)dimethylsilanetitanium dichloride
(phenylamido)(tetramethyl-η⁵-cyclopentadienyl)dimethylsilanetitanium dichloride
(cyclohexylamido)(tetramethyl-η⁵-cyclopentadienyl)dimethylsilanetitanium dichloride
(t-butylamido)(1-η⁵-indenyl)dimethylsilanetitanium dichloride
(phenylamido)(1-η⁵-indenyl)dimethylsilanetitanium dichloride
(phenylamido)(1-η⁵-indenyl)dimethylsilanetitanium dichloride
(cyclohexylamido)(1-η⁵-indenyl)dimethylsilanetitanium dichloride
(t-butylamido)(9-η⁵-fluorenyl)dimethylsilanetitanium dichloride
(phenylamido)(9-η⁵-fluorenyl)dimethylsilanetitanium dichloride
(cyclohexylamido)(9-η⁵-fluorenyl)dimethylsilanetitanium dichloride.

5. The process as claimed in one or more of claims 1 to 4, wherein the cocatalyst used is an aluminoxane.

6. The process as claimed in one or more of claims 1 to 5, by polymerization of from 0.1 to 99.9% by weight, based on the total amount of monomers, of at least one polycyclic olefin of the formula III, IV, IV', V, VI, VII or VIII 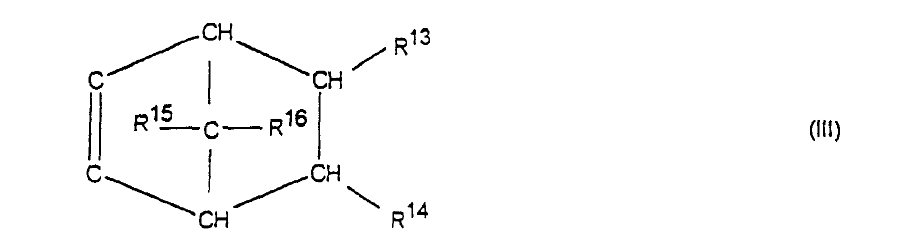 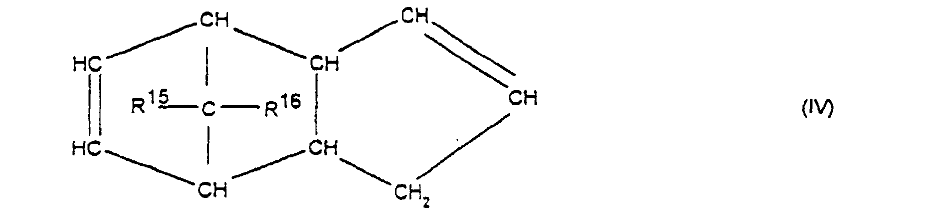 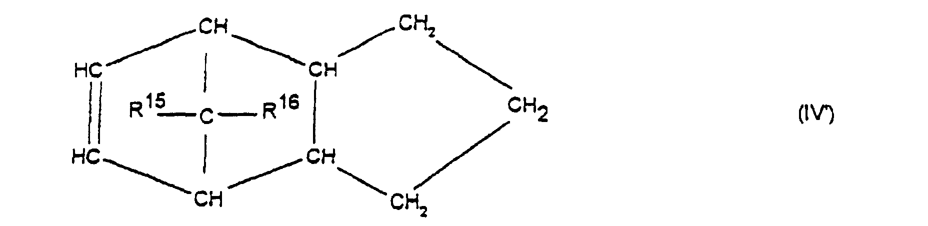 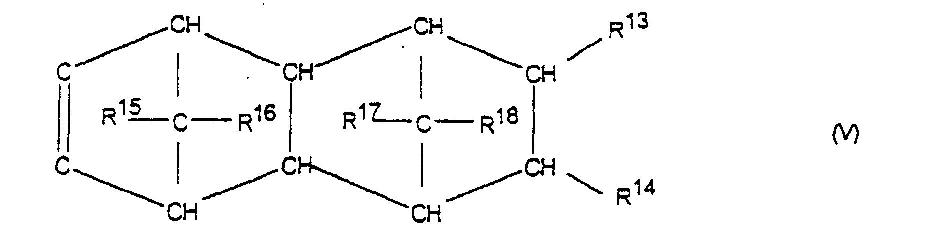 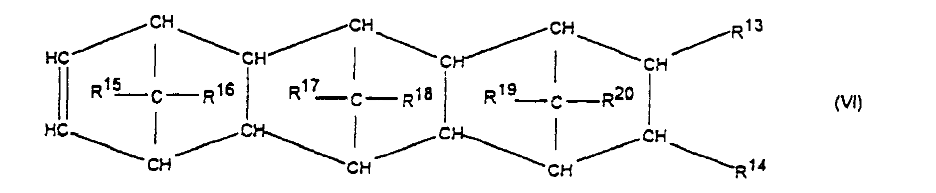 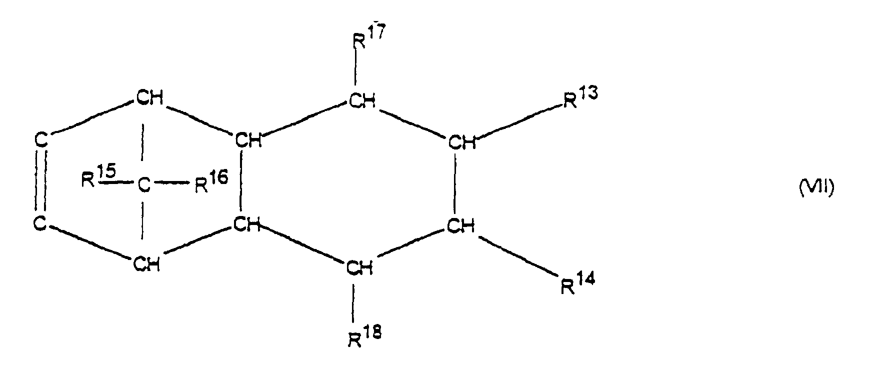 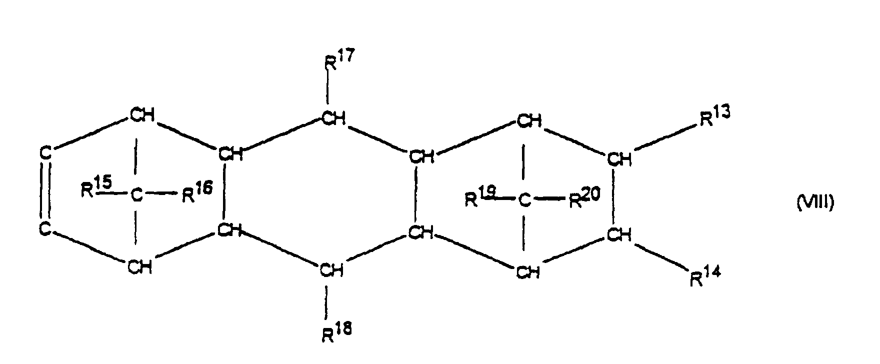 where R¹³, R¹⁴, R¹⁵, R¹⁶, R¹⁷, R¹⁸, R¹⁹ and R²⁰ are identical or different and are each a hydrogen atom or a hydrocarbon radical, where identically numbered radicals in the various formulae can have different meanings, from 0 to 99.9% by weight, based on the total amount of monomers, of at least one monocyclic olefin of the formula IX 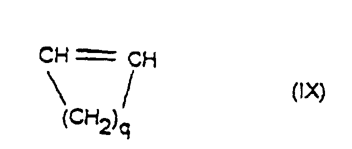 where q is from 2 to 10, and from 0.1 to 99.9% by weight, based on the total amount of monomers, are of at least one acyclic 1-olefin of the formula X 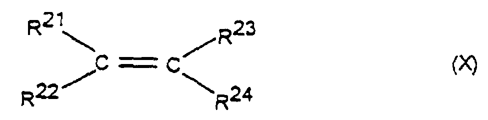 where R²¹, R²², R²³ and R²⁴ are identical or different and are each a hydrogen atom or a hydrocarbon radical, preferably a C₆-C₁₀-aryl radical or a C₁₋C₈-alkyl radical.

7. The process as claimed in one or more of claims 1 to 6, wherein the polycyclic olefin is a compound of the formula III or V in which R¹³, R¹⁴, R¹⁵, R¹⁶, R¹⁷, R¹⁸, R¹⁹ and R²⁰ are identical or different and are each a hydrogen atom or a hydrocarbon radical, in particular a C₆-C₁₀-aryl radical or a C₁-C₈-alkyl radical, where identically numbered radicals in the various formulae can have different meanings.

8. The process as claimed in one or more of claims 1 to 7, wherein the polycyclic olefin is norbornene or tetracyclododecane.

9. The process as claimed in one or more of claims 1 to 8, wherein the acyclic 1-olefin is ethylene.

10. The process as claimed in one or more of claims 1 to 9, wherein the cycloolefin copolymer comprises at least 5 mol% of units derived from the cycloolefin.

11. The process as claimed in one or more of claims 1 to 10, wherein the cycloolefin copolymer consists of units derived from monomers which have, apart from the olefinic double bond required for the polymerization, no further unsaturated functionalities.

12. The process as claimed in one or more of claims 1 to 11, wherein a temperature of from -78 to 150°C and a pressure of from 0.01 to 64 bar are employed.

13. The process as claimed in one or more of claims 1 to 12, wherein a temperature of from 0 to 100°C and a pressure of from 0.01 to 64 bar are employed.

14. The process as claimed in one or more of claims 1 to 13, wherein the polymerization is carried out in the liquid cycloolefin itself or in a cycloolefin solution.

15. The process as claimed in one or more of claims 1 to 14, wherein the preactivation time is from 5 to 60 minutes.

16. A cycloolefin copolymer obtainable by polymerization of at least one polycyclic olefin, if desired at least one monocyclic olefin and at least one acyclic 1-olefin, with the incorporated proportions of cyclic compounds being from 15 to 60 mol% in the presence of a catalyst system comprising at least one cocatalyst and at least one metal complex having a strained geometry and the formula (I) 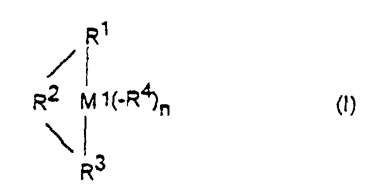 where
M¹ is a metal of groups 3 to 10 or of the lanthanide series of the Periodic Table of the Elements,
R¹ is a delocalized acyclic π system such as C₄-C₂₀-alkenyl, C₄-C₂₀-alkynyl, C₃-C₂₀-allyl, C₄-C₂₀-alkadienyl, C₄-C₂₀-polyenyl or a comparable structure which may comprise up to 5 hetero atoms, or an unsubstituted or substituted delocalized C₅-C₄₀-cyclic π system or a comparable structure which may comprise up to 5 hetero atoms,
R² is a single- or multi-membered bridge which links the radicals R¹ and R³ and comprises at least one atom of group 14 of the Periodic Table of the Elements or at least one boron atom and may comprise one or more sulfur or oxygen atoms and can form a fused ring system together with R¹,
R³ is an anionic or nonionic ligand which is coordinated to M¹ and comprises one or more nitrogen, phosphorus, oxygen and/or sulfur atoms and can form a fused ring system together with R², and
R⁴ is an anionic or nonionic ligand, where n = 0, 1, 2, 3 or 4 depending on the valence of M.

17. The use of a preactivated catalyst system comprising a metal complex having the formula (I) as claimed in claim 1 for preparing a cycloolefin copolymer.

18. The use of a cycloolefin copolymer as claimed in one or more of claim 16 for producing moldings such as extruded parts such as films, sheets, hoses, pipes, rods and fibers or injection-molded parts of any shape and size.

## Revendications

1. Procédé pour la préparation d'un copolymère cyclooléfinique par polymérisation de 0,1 à 99,9% en poids, par rapport à la quantité totale des monomères, d'au moins une oléfine polycyclique, de 0 à 99,9% en poids, par rapport à la quantité totale des monomères, d'au moins une oléfine monocyclique et de 0,1 à 99,9% en poids, par rapport à la quantité totale des monomères, d'au moins une 1-oléfine acyclique, en présence d'un système catalyseur qui est constitué d'au moins un cocatalyseur et d'au moins un complexe métallique de coordination à géométrie tendue de formule (I) 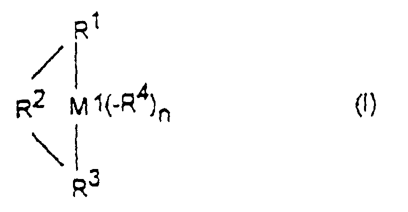
M¹ représentant un métal du groupe 3 à 10 ou de la série des lanthanides du système périodique des éléments,
R¹ signifiant un système π acyclique délocalisé tel qu'un alcényle en C₄ à C₂₀, un alcynyle en C₄ à C₂₀, un allyle en C₃ à C₂₀, un alcadiényle en C₄ à C₂₀, un polyényle en C₄ à C₂₀ ou des structures comparables, qui peuvent contenir jusqu'à 5 hétéroatomes, ou un système π cyclique en C₅ à C₄₀ délocalisé non substitué ou substitué ou des systèmes comparables qui peuvent contenir jusqu'à 5 hétéroatomes,
R² représentant un pont à un ou plusieurs membres, qui relie les radicaux R¹ et R³ et qui contient au moins un atome du groupe 14 du système périodique des éléments ou au moins un atome de bore et qui peut contenir un ou plusieurs atomes de soufre ou d'oxygène et qui peut former un système cyclique condensé avec R¹,
R³ signifiant un ligand anionique ou non ionique, qui est coordonné à M¹ et qui contient un ou plusieurs atomes d'azote, de phosphore, d'oxygène et/ou de soufre et qui peut former avec R² un système cyclique condensé et
R⁴ représentant un ligand anionique ou non ionique, n signifiant 0, 1, 2, 3 ou 4 en fonction de la valence de M, **caractérisé en ce que** le complexe de coordination métallique est activé au préalable avant l'utilisation dans la réaction de polymérisation avec le cocatalyseur, sous réserve que des complexes métalliques de coordination, dans lesquels R¹ est un ligand monocyclopentadiényle asymétriquement substitué et le composé diméthylsilyl(tétraméthylcyclopentadiényl)-(tert-butylamido)diméthylhafnium aient été exclus.

2. Procédé selon la revendication 1, le complexe de coordination métallique signifiant un composé de formule (Ia) : 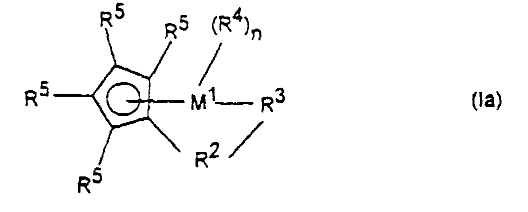
M¹ représentant un métal du groupe 4 ou de la série des lanthanides du système périodique des éléments,
R² représentant un pont à un ou plusieurs membres, qui relie le système π cyclique coordonné en η⁵ et R³ et signifiant de préférence 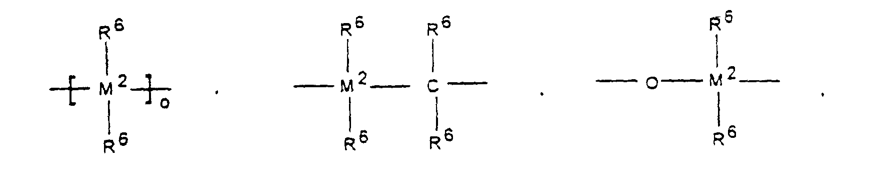 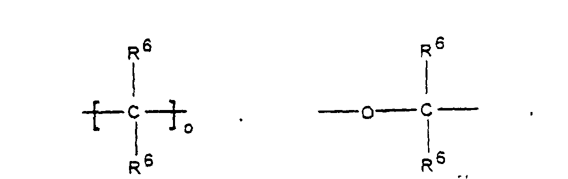 =BR⁶, =AlR⁶, -Ge-, -Sn-, -O-, -S-, =SO, =SO₂, =NR⁶, =CO, =PR⁶ ou =P(O)R⁶, R⁶ étant identiques ou différents et représentant un atome d'hydrogène, un atome d'halogène, un groupement carboné en C₁ à C₄₀, tel qu'un groupement alkyle en C₁ à C₁₀ qui peut avoir été halogéné, une groupement aryle en C₆ à C₂₀ qui peut avoir été halogéné, un groupement aryloxy en C₆ à C₂₀, alcényle en C₂ à C₁₂, arylalkyle en C₇ à C₄₀, alkylaryle en C₇ à C₄₀, arylalcényle en C₈ à C₄₀, -SiR⁷₃, -NR⁷₂, -Si(OR⁷)₃, -Si(SR⁷)₃ ou -PR⁷₂, groupements
dans lesquels R⁷ sont identiques ou différents et représente un atome d'halogène, un groupement alkyle en C₁ à C₁₀ ou un groupement aryle en C₆ à C₁₀ ou forment un système cyclique, o étant ≥ 1, et
M² représentant un silicium, un germanium ou un étain,
R³ représentant O, S, NR⁸, PR⁸ ou un ligand neutre donneur de 2 électrons, choisi parmi le groupe OR , SR⁸, NR⁸₂, PR⁸₂, R⁸ signifiant un atome d'hydrogène, un atome d'halogène, un groupement carboné en C₁ à C₄₀ tel qu'un groupement alkyle en C₁ à C₈ qui peut avoir été halogéné, une groupement aryle en C₆ à C₂₀ qui peut avoir été halogéné, un groupement aryloxy en C₆ à C₂₀, alcényle en C₂ à C₁₂, arylalkyle en C₇ à C₄₀, alkylaryle en C₇ à C₄₀, arylalcényle en C₈ à C₄₀, -SiR⁹₃, -NR⁹₂, -Si(OR⁹)₃, -Si(SR⁹)₃ ou -PR⁹₂, groupements dans lesquels R⁹ peuvent être identiques ou différents et représentent un atome d'halogène, un groupement alkyle en C₁ à C₈ ou un groupement aryle en C₆ à C₁₀,
R⁴ étant identiques ou différents et représentant un atome d'hydrogène, un groupement carboné en C₁ à C₄₀, tel qu'un groupement alkyle en C₁ à C₁₀, alcoxy en C₁ à C₁₀, aryle en C₆ à C₁₀, aryloxy en C₆ à C₂₅, alcényle en C₂ à C₁₀, arylalkyle en C₇ à C₄₀ ou arylalcényle en C₇ à C₄₀, un groupement OH, un atome d'halogène ou un groupement NR¹⁰₂, dans lequel R¹⁰ représentent un atome d'halogène, un groupement alkyle en C₁ à C₁₀, ou un groupement aryle en C₆ à C₁₀ ou R⁴ formant un système cyclique avec les atomes qui les lient, n signifiant 1 ou 2,
R⁵ étant identiques ou différents et représentant un atome d'hydrogène, un atome d'halogène, un groupement carboné en C₁ à C₄₀, tel qu'un groupement alkyle en C₁ à C₁₀ qui peut avoir été halogéné, un groupement aryle en C₆ à C₂₀ qui peut avoir été halogéné, un groupement aryloxy en C₆ à C₂₀, alcényle en C₂ à C₁₂, arylalkyle en C7 à C₄₀, alkylaryle en C₇ à C₄₀ ou arylalcényle en C₈ à C₄₀, -SiR¹¹₃, -NR¹¹₂, -Si(OR¹¹)₃, -Si(SR¹¹)₃ ou -PR¹¹₂, groupements dans lesquels R¹¹ peuvent être identiques ou différents et représentent un atome d'halogène, un groupement alkyle en C₁ à C₁₀ ou un groupement aryle en C₆ à C₁₀ ou forment un système cyclique, ou deux ou plusieurs substituants R⁵ voisins formant un système cyclique avec les atomes qui les lient, qui contient de préférence 4 à 40, de manière particulièrement préférée 6 à 20, atomes de carbone.

3. Procédé selon l'une ou plusieurs des revendications 1 à 2, le complexe métallique de coordination représentant un composé de formule (Ib) : 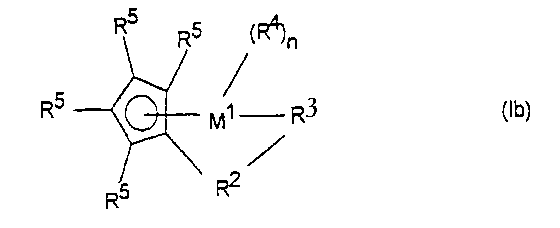
M¹ signifiant un titane
R² représentant un pont à un, deux ou trois membres, qui relie le système π cyclique coordonné en η⁵ et R³ et signifiant de préférence 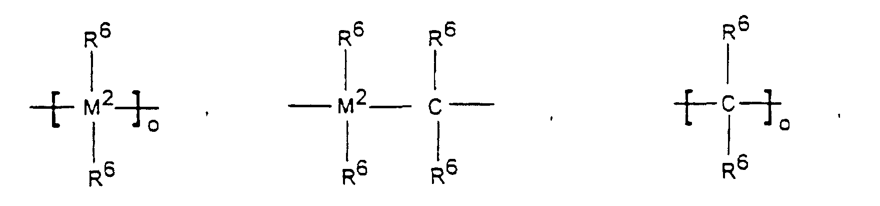
R⁶ étant identique ou différent et représentant un atome d'hydrogène, un groupement carboné en C₁ à C₄₀, tel qu'un groupement alkyle en C₁ à C₁₀ qui peut avoir été halogéné, un groupement aryle en C₆ à C₂₀ qui peut avoir été halogéné, un groupement aryloxy en C₆ à C₂₀, alcényle en C₂ à C₁₂, arylalkyle en C₇ à C₄₀, alkylaryle en C₇ à C₄₀ ou arylalcényle en C₈ à C₄₀, o signifiant 1, 2 ou 3 et
M² représentant un silicium,
R³ représentant NR⁸, R⁸ représentant un atome d'hydrogène, un groupement carboné en C₁ à C₄₀, tel qu'un groupement alkyle en C₁ à C₈ qui peut avoir été halogéné, un groupement alcoxy en C₁ à C₁₀, un groupement aryle en C₆ à C₂₀ qui peut avoir été halogéné, un groupement aryloxy en C₆ à C₂₀, alcényle en C₂ à C₁₂, arylalkyle en C₇ à C₄₀, alkylaryle en C₇ à C₄₀ ou arylalcényle en C₈ à C₄₀,
R⁴ pouvant être identiques ou différents et représentant un atome d'hydrogène, un groupement carboné en C₁ à C₄₀, tel qu'un groupement alkyle en C₁ à C₁₀, alcoxy en C₁ à C₁₀, aryle en C₆ à C₁₀, aryloxy en C₆ à C₂₅, alcényle en C₂ à C₁₀, arylalkyle en C₇ à C₄₀ ou arylalcényle en C₇ à C₄₀, un groupement OH, un atome d'halogène ou un groupement NR¹⁰₂, dans lequel R¹⁰ représente un atome d'halogène, un groupement alkyle en C₁ à C₁₀ ou un groupement aryle en C₆ à C₁₀, ou formant un système cyclique avec les atomes qui les lient
R⁵ étant identiques ou différents et représentant un atome d'hydrogène, un groupement alkyle en C₁ à C₁₀ ou triméthylsilyle ou deux des substituants R⁵ formant un cycle condensé aromatique à six membres avec le système cyclopentadiényle qui les lie.

4. Procédé selon l'une ou plusieurs des revendications 1 à 3, le complexe métallique de coordination de formule (I) étant un composé choisi parmi les composés
dichlorure de (t-butylamido)(tétraméthyl-η⁵-cyclopentadiényl)diméthylsilanetitane,
dichlorure de (phénylamido)(tétraméthyl-η⁵-cyclopentadiényl)diméthylsilanetitane,
dichlorure de (cyclohexylamido)(tétraméthyl-η⁵-cyclopentadiényl)diméthylsilanetitane,
dichlorure de (t-butylamido)(1-η⁵-indényl)diméthylsilanetitane,
dichlorure de (phénylamido)(1-η⁵-indényl)diméthylsilanetitane,
dichlorure de (phénylamido)(1-η⁵-indényl)diméthylsilanetitane,
dichlorure de (cyclohexylamido)(1-η⁵-indényl)diméthylsilanetitane,
dichlorure de (t-butylamido)(9-η⁵-fluorényl)diméthylsilanetitane,
dichlorure de (phénylamido)(9-η⁵-fluorényl)diméthylsilanetitane,
dichlorure de (cyclohexylamido) (9-η⁵-fluorényl)diméthylsilanetitane.

5. Procédé selon l'une ou plusieurs des revendications 1 à 4, dans lequel un aluminoxane est utilisé comme cocatalyseur.

6. Procédé selon l'une ou plusieurs des revendications 1 à 5, par polymérisation de 0,1 à 99,9% en poids, par rapport à la quantité totale des monomères, d'au moins une oléfine polycyclique de formules III, IV, IV', V, VI, VII ou VIII 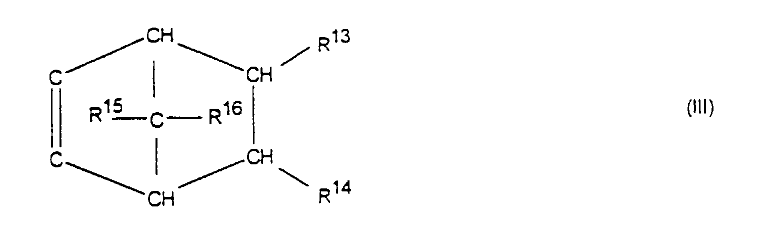 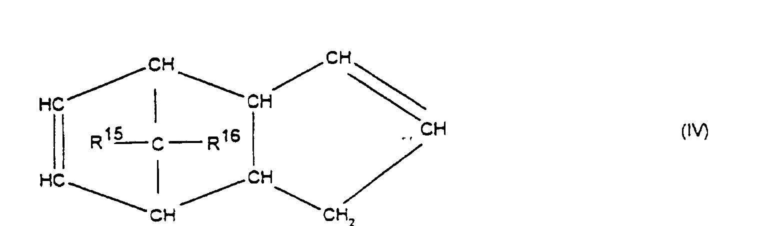 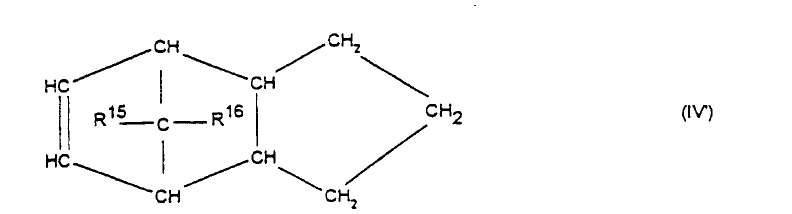 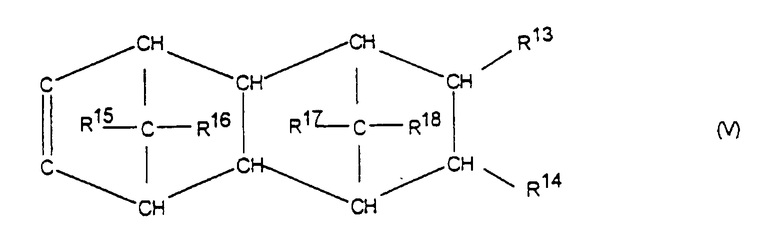 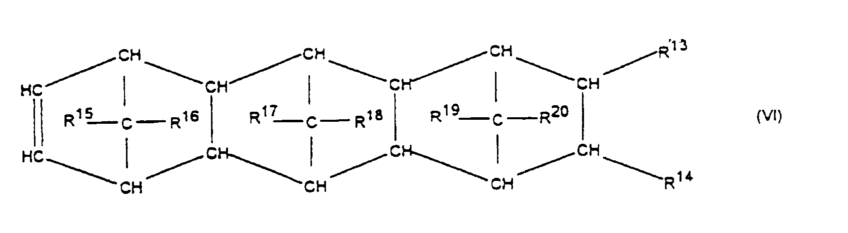 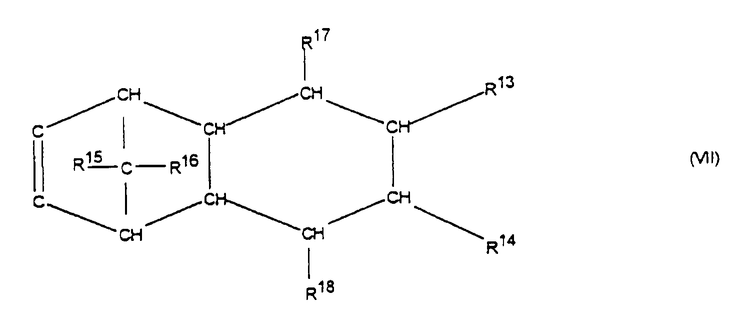 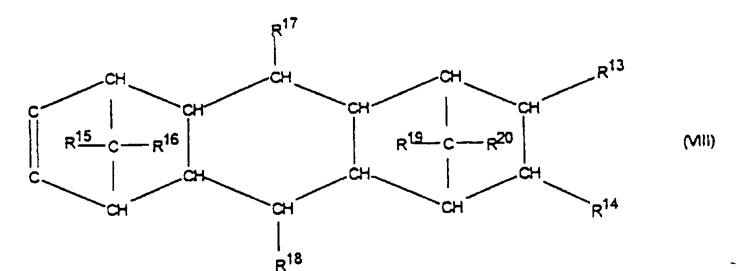 dans lesquelles R¹³, R¹⁴, R¹⁵, R¹⁶, R¹⁷, R¹⁸, R¹⁹ et R²⁰ sont identiques ou différents et signifient un atome d'hydrogène ou un radical hydrocarboné, des radicaux identiques dans les différentes formules pouvant avoir une signification différente, de 0 à 99,9% en poids, par rapport à la quantité totale des monomères, d'au moins une oléfine monocyclique de formule IX 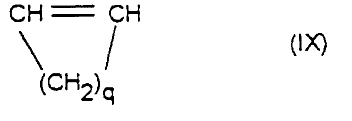 dans laquelle q représente un nombre de 2 à 10, et de 0,1 à 99,9% en poids, par rapport à la quantité totale des monomères, d'au moins une 1-oléfine acyclique de formule X 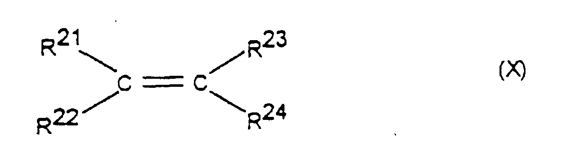 dans laquelle R²¹, R²², R²³ et R²⁴ sont identiques ou différents et signifient un atome d'hydrogène ou un radical hydrocarboné, de préférence un radical aryle en C₆ à C₁₀ ou un radical alkyle en C₁ à C₈.

7. Procédé selon l'une ou plusieurs des revendications 1 à 6, l'oléfine polycyclique étant un composé de formules III ou V, dans lesquelles R¹³, R¹⁴, R¹⁵ R¹⁶, R¹⁷, R¹⁸, R¹⁹ et R²⁰ sont identiques ou différents et signifient un atome d'hydrogène ou un radical hydrocarboné, en particulier un radical aryle en C₆ à C₁₀ ou un radical alkyle en C₁ à C₈, des radicaux identiques dans les différentes formules pouvant avoir une signification différente.

8. Procédé selon l'une ou plusieurs des revendications 1 à 7, l'oléfine polycyclique étant le norbornène ou le tétracyclododécène.

9. Procédé selon l'une ou plusieurs des revendications 1 à 8, la 1-oléfine acyclique étant l'éthylène.

10. Procédé selon l'une ou plusieurs des revendications 1 à 9, le copolymère cyclooléfinique contenant au moins 5% en mole d'unités dérivées de la cyclooléfine.

11. Procédé selon l'une ou plusieurs des revendications 1 à 10, le copolymère cyclooléfinique étant constitué d'unités dérivées de monomères qui ne présentent pas d'autres fonctionnalités insaturées en dehors de la double liaison oléfinique nécessaire pour la polymérisation.

12. Procédé selon l'une ou plusieurs des revendications 1 à 11, dans lequel on règle une température de -78°C à 150°C et une pression de 0,01 à 64 bars.

13. Procédé selon l'une ou plusieurs des revendications 1 à 12, dans lequel on règle une température de 0 à 100°C et une pression de 0,01 à 64 bars.

14. Procédé selon l'une ou plusieurs des revendications 1 à 13, la polymérisation étant réalisée dans la cyclooléfine liquide même ou dans une solution de cyclooléfine.

15. Procédé selon l'une ou plusieurs des revendications 1 à 14, la durée d'activation préalable étant de 5 à 60 minutes.

16. Copolymère cyclooléfinique obtenu par polymérisation d'au moins une oléfine polycyclique, le cas échéant d'au moins une oléfine monocyclique et d'au moins une 1-oléfine acyclique, le taux d'incorporation des composants cycliques étant de 15 à 60% en mole, en présence d'un système catalyseur qui est constitué d'au moins un cocatalyseur et d'au moins un complexe métallique de coordination à géométrie tendue de formule (I) 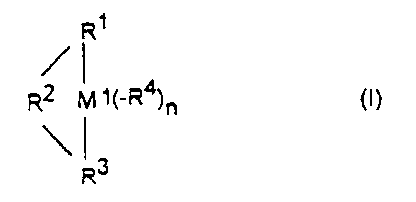
M¹ représentant un métal du groupe 3 à 10 ou de la série des lanthanides du système périodique des éléments,
R¹ signifiant un système π acyclique délocalisé tel qu'un alcényle en C₄ à C₂₀, un alcynyle en C₄ à C₂₀, un allyle en C₃ à C₂₀, un alcadiényle en C₄ à C₂₀, un polyényle en C₄ à C₂₀, ou des structures comparables, qui peuvent contenir jusqu'à 5 hétéroatomes, ou un système π cyclique en C₅ à C₄₀ délocalisé non substitué ou substitué ou des systèmes comparables qui peuvent contenir jusqu'à 5 hétéroatomes,
R² représentant un pont à un ou plusieurs membres, qui relie les radicaux R¹ et R³ et qui contient au moins un atome du groupe 14 du système périodique des éléments ou au moins un atome de bore et qui peut contenir un ou plusieurs atomes de soufre ou d'oxygène et qui peut former avec R¹ un système cyclique condensé,
R³ signifiant un ligand anionique ou non ionique, qui est coordonné à M¹ et qui contient un ou plusieurs atomes d'azote, de phosphore, d'oxygène et/ou de soufre et qui peut former avec R² un système cyclique condensé et
R⁴ représentant un ligand anionique ou non ionique, n signifiant 0, 1, 2, 3 ou 4 en fonction de la valence de M.

17. Utilisation d'un système catalyseur activé au préalable contenant un complexe métallique de coordination présentant la formule (I) selon la revendication 1 pour la préparation d'un copolymère cyclooléfinique.

18. Utilisation d'un copolymère cyclooléfinique selon la revendication 16 pour la fabrication de corps façonnés, tels que des pièces extrudées telles que des films, des plaques, des flexibles, de tuyaux, des tubes, des barres et des fibres ou des articles moulés par injection de forme et de taille quelconque.
